Europäisches Patentamt

European Patent Office

Office européen des brevets

(19)

(11) **EP 1 600 201 A1**

(12) **EUROPEAN PATENT APPLICATION**
published in accordance with Art. 158(3) EPC

(43) Date of publication:
**30.11.2005 Bulletin 2005/48**

(51) Int Cl.⁷: **B01D 46/10**, B01D 46/54,
B03C 3/02, B03C 3/14,
B03C 3/45, A61L 9/16,
A61L 9/00, A61L 9/18,
A01N 25/34, A01N 43/16,
A01N 59/16, A01N 63/00,
A01N 65/00

(21) Application number: **04714940.6**

(22) Date of filing: **26.02.2004**

(86) International application number:
**PCT/JP2004/002321**

(87) International publication number:
**WO 2004/078320 (16.09.2004 Gazette 2004/38)**

(84) Designated Contracting States:
**AT BE BG CH CY CZ DE DK EE ES FI FR GB GR
HU IE IT LI LU MC NL PT RO SE SI SK TR**
Designated Extension States:
**AL LT LV MK**

(30) Priority: **04.03.2003 JP 2003057720
10.11.2003 JP 2003379404
21.11.2003 JP 2003393039**

(71) Applicant: **DAIKIN INDUSTRIES, LTD.**
**Osaka-shi, Osaka 530-8323 (JP)**

(72) Inventors:
• **OKAMOTO, Yoshio, C/o Daikin Industries, Ltd.**
  **Kusatsu-shi, Shiga 525-0044 (JP)**
• **TAIRA, Shigeharu, C/o Daikin Industries, Ltd.**
  **Kusatsu-shi, Shiga 525-0044 (JP)**
• **KURODA, Tarou, C/o Daikin Industries, Ltd.**
  **Kusatsu-shi, Shiga 525-0044 (JP)**
• **NAKADA, Satoki, C/o Daikin Industries, Ltd.**
  **Osaka-shi, Osaka 530-8323 (JP)**

(74) Representative: **Goddar, Heinz J.**
**FORRESTER & BOEHMERT**
**Pettenkoferstrasse 20-22**
**80336 München (DE)**

(54) **AIR CLEANING MEMBER, AIR CLEANING UNIT AND AIR CONDITIONER**

(57) The present invention provides an air purification member, an air purification unit and an air conditioning apparatus that can reduce the generation of unpleasant odor or the occurrence of air contamination without necessitating frequent cleaning or replacement. A first photocatalytic filter (33) of an air purifier is a filter for purifying air that contains particulates containing viruses or microbes, and is provided with a photocatalytic filter (331) and a photocatalytic apatite (334). The photocatalytic filter (331) collects the particulates. The photocatalytic apatite (334) removes viruses or microbes that are contained in the particulates collected by the photocatalytic filter (331).

Fig. 2

## Description

### Technical Field

The present invention relates to air purification members, air purification units and air conditioning apparatuses.

### Background Art

Conventionally, air conditioning apparatuses are known which improve the degree of comfort inside a room by supplying conditioned air into the room in a building or a residence, for example. For example, an air purifier can maintain a comfortable indoor environment by blowing purified air into a room (e.g., see JP H11-319451A).

Such an air purifier includes, for example, a casing, a blowing device, a pre-filter and an air purification member. The casing has an intake port for drawing in indoor air and a discharge port for discharging purified air into the room. The blowing device draws indoor air from the intake port into the casing, and discharges purified air into the room. The pre-filter is provided so as to cover the intake port, and removes, from the air, dust or dirt particles having a relatively large diameter that are contained in the air drawn into the casing. The air that has passed through the pre-filter passes through the air purification member before it is delivered into the room. In the air purification member, dust and dirt particles or the like having a relatively small diameter that cannot be removed by the pre-filter are removed from the air.

In this air purifier, indoor air is drawn into the casing by the blowing device. At this time, dust and dirt particles or the like having a relatively large diameter in the air are removed from the air in the pre-filter. Then, the air that has passed through the pre-filter passes through the air purification member. At this time, dust and dirt particles or the like having a relatively small diameter are removed from the air in the air purification member. Thereafter, the air that has passed through the air purification member is blown into the room by the blowing device. Thus, the air purifier supplies purified air into the room.

In such an air purifier, particles that have been removed from the air adhere to the filter. These particles include dust and dirt particles or the like contained in the air. Further, the dust or dirt particles contain microbes such as fungi and bacteria, and/or viruses. However, in conventional air purifiers, these microbes or viruses are left on the filter during the period in which the particles are removed and the filter is cleaned or replaced. Accordingly, the microbes may proliferate on the filter, or the viruses may be released again, thereby possibly causing unpleasant odor or air contamination. For this reason, in the case of conventional air purifiers, for example, the users of the air purifiers need to perform frequent cleaning or replacement of the filter, in order to prevent the generation of unpleasant odor or air contamination.

### Disclosure of Invention

It is an object of the present invention to provide a filter and an air conditioning apparatus that can reduce the generation of unpleasant odor or the occurrence of air contamination without the need of frequent cleaning or replacement.

An air purification member described in claim 1 is an air purification member for purifying air that contains particulates containing viruses or microbes, including: a dust collection portion; and a microbe removal portion. The dust collection portion collects the particulates. The microbe removal portion removes the viruses or the microbes contained in the particulates that are collected by the dust collection portion. It should be noted that "dust collection portion" as mentioned herein refers to, for example, filters (e.g., an electrostatic filter (e.g., a filter in which substances having a positive charge and a negative charge are carried on fiber constituting nonwoven fabric), a filtration type filter, an impact adhesive type filter, an adsorption type filter and an absorption type filter), adsorbents (e.g., activated carbon, zeolite and apatite) and electric dust collectors.

In this air purification member, particulates are collected by the dust collection portion. Then, viruses or microbes that are contained in the particulates collected by the dust collection portion are removed by the microbe removal portion.

In the case of conventional air purification members, the collected viruses or microbes are left on the air purification member for a long period of time. However, in the above-mentioned air purification member, viruses or microbes that are collected by the dust collection portion are removed by the microbe removal portion. Accordingly, this air purification member can reduce the generation of unpleasant odor or the occurrence of air contamination without the need of frequent cleaning or replacement.

An air purification member described in claim 2 is the air purification member according to claim 1, wherein the dust collection portion carries thereon the microbe removal portion. Here, the microbe removal portion is carried on the dust collection portion. Accordingly, this air purification member can readily remove viruses or microbes.

An air purification member described in claim 3 is the air purification member according to claim 2, wherein the microbe removal portion includes a photocatalyst. Here, the microbe removal portion includes a photocatalyst. Accordingly, when light of an appropriate wavelength region is radiated to this photocatalyst, the photocatalyst removes viruses or microbes that are collected in the dust collection portion. Consequently, this air purification member can readily remove viruses or microbes.

An air purification member described in claim 4 is the air purification member according to claim 1, wherein the microbe removal portion includes a photocatalyst. The photocatalyst is included in the dust collection portion.

Here, the microbe removal portion includes a photocatalyst that is included in the dust collection portion. Accordingly, viruses or microbes that are collected in the dust collection portion are removed by the photocatalyst included in the dust collection portion. Consequently, this air purification member can readily remove viruses or microbes.

An air purification member described in claim 5 is the air purification member according to claim 3 or 4, wherein the photocatalyst is a visible light photocatalyst.

Here, the photocatalyst is a visible light photocatalyst. Accordingly, it is possible to remove viruses or microbes with the use of the photocatalyst in any locations where visible light can be obtained, without the need of preparing a special light source. Consequently, this air purification member can remove viruses or microbes with a simple configuration. In addition, if this air purification member is attached in the vicinity of the inlet port of an air conditioning apparatus so as to allow irradiation of external light, then the performance of this air purification member can be more efficiently exploited.

An air purification member described in claim 6 is the air purification member according to claim 3 or 4, wherein the photocatalyst is apatite having photocatalytic activity.

Conventionally, for example, a mixture of an adsorbent, such as zeolite, and titanium oxide is used as a photocatalyst. On the other hand, apatite is known to have a high adsorption property with respect to viruses or microbes, and it is conceivable to adopt a configuration in which apatite is used in place of the adsorbent in the above-described mixture, in order to increase the effect of the microbe removal portion. However, even if adopted, such a configuration is only effective for viruses or microbes that are adsorbed in the vicinity of titanium oxide, which exhibits catalytic activity. When viruses or microbes are adsorbed in apatite, without the presence of titanium oxide in the vicinity thereof, they cannot be removed and thus remain on the apatite, thus possibly causing the generation of unpleasant odor or the occurrence of air contamination after a long period of time has elapsed. However, since the above-mentioned apatite having photocatalytic activity has photocatalyst activity in its adsorption site, it is possible to remove the adsorbed viruses or microbes substantially completely. Consequently, this air purification member can reduce the generation of unpleasant odor or the occurrence of air contamination without necessitating frequent cleaning or replacement.

An air purification member described in claim 7 is the air purification member according to any of claims 3 to 5, wherein the photocatalyst is a mixed photocatalyst. Additionally, in the mixed photocatalyst, the apatite having photocatalytic activity and a photocatalytic material are mixed.

Although apatite-based photocatalysts have higher adsorption ability with respect to viruses or microbes than conventionally used ones such as titanium oxide, they may not be able to obtain sufficient reactivity depending on the conditions of the light source. On the other hand, in the case of commonly used photocatalytic materials such as metal oxide photocatalysts or carbon-based photocatalysts, the dominant wavelength region of light in which catalytic reaction takes place can be easily changed, for example, by control of the crystal structure or by ion injection, so that they exhibit relatively high reactivity, regardless of the conditions of the light source. Accordingly, such a mixed photocatalyst can remove viruses or microbes that are adsorbed by apatite located in the vicinity of a commonly used photocatalyst such as a metal oxide photocatalyst or a carbon-based photocatalyst, even if the light source is in poor conditions.

It should be noted that "apatite having photocatalytic activity" as mentioned herein refers to, for example, a calcium hydroxyapatite to which photocatalytic activity is imparted by replacing the calcium ions with titanium ions. Since such apatite is not inherently a photocatalytic material, it is difficult to control the dominant wavelength region of light in which catalytic reaction takes place. Further, "photocatalytic material" as mentioned herein refers to substances having photocatalytic activity as one of their inherent properties. A typical example of the photocatalytic material is titanium oxide, and other examples include metal oxide photocatalysts, carbon-based photocatalysts, and nitrides and oxynitrides that are made up of transition metals. Examples of the metal oxide photocatalysts include strontium titanate, zinc oxide, tungsten oxide and iron oxide. Further, examples of the carbon-based photocatalysts include fullerene such as $C_{60}$. These photocatalytic materials can be made responsive to visible light by changing their band gap by ion injection, for example.

An air purification member described in claim 8 is the air purification member according to any of claims 3 to 7, wherein the dust collection portion is formed by fiber. Additionally, this fiber is constituted by a core and a covering layer. Further, this covering layer holds the photocatalyst in such a manner that a portion of the photocatalyst is exposed to the air side.

In general, a photocatalyst is carried on fiber using a method such as performing injection molding, with a powder or the like of the photocatalyst dispersed in a resin. However, such fiber gradually deteriorates each time it is irradiated with light having a wavelength to which that photocatalyst produces catalytic reaction. Accordingly, an air purification member that is constituted by such fiber will gradually lose its strength. Furthermore, there is also a problem in that an object that is made of resin containing foreign substances generally tends to be fragile.

However, here, the fiber has the core, and the photocatalyst is carried only on the covering layer. Accordingly, although the covering layer gradually deteriorates each time it is irradiated with light having a wavelength to which the photocatalyst produces catalytic reaction, the core will not deteriorate owing to the light. Moreover, this core has favorable strength, since no foreign substance (photocatalyst) is mixed into the core. Accordingly, this air purification member can maintain the strength of the dust collection portion for a long period of time.

Furthermore, here, the covering layer holds the photocatalyst in such a manner that a portion of the photocatalyst is exposed to the air side. This allows the photocatalyst to come into contact with viruses or microbes. Accordingly, it is possible to remove viruses or microbes.

In addition, it is preferable that the material of the core and the material of the covering layer may be those of a combination having excellent adhesion. It is also preferable that the thickness of the covering layer is sufficiently smaller than that of the core.

An air purification member described in claim 9 is the air purification member according to claim 4 or 5, wherein the apatite is included in the dust collection portion.

Here, the apatite is included in the dust collection portion. In general, apatite is known to have an excellent effect of adsorbing fungi, bacteria, viruses, ammonias, nitrogen oxides and aldehydes, for example. Accordingly, even more particulates containing viruses or microbes can be collected in the dust collection portion. That is to say, this air purification member can remove even more viruses or microbes, thus making it possible to further reduce the generation of unpleasant odor or the occurrence of air contamination.

An air purification member described in claim 10 is the air purification member according to claim 9, wherein the apatite is provided on an upstream surface of the dust collection portion with respect to a direction of air flow.

In general, viruses or microbes tend to adhere to dust or dirt particles. Then, most dust or dirt particles are usually blocked on the upstream surface of the dust collection portion in the direction of air flow. Accordingly, more viruses or microbes are supposed to be present on the upstream surface of the dust collection portion in the direction of air flow. Here, the apatite is provided on the upstream surface of the dust collection portion in the direction of air flow. Consequently, in this air purification member, viruses or microbes can be efficiently adsorbed by the apatite.

An air purification member described in claim 11 is the air purification member according to claim 9, wherein a light source is disposed in a space located downstream of the dust collection portion. Additionally, the apatite and the photocatalyst are provided on a downstream surface of the dust collection portion with respect to a direction of air flow.

In general, a light source is often provided upstream of the dust collection portion, where the collection concentration of dust or dirt particles is high. However, here, the light source is disposed in a space located downstream of the dust collection portion. Further, the apatite and the photocatalyst are provided on the downstream surface of the dust collection portion in the direction of air flow. Accordingly, air containing viruses or microbes that have not been collected in the dust collection portion comes in contact with the apatite and the photocatalyst. As a result, the apatite adsorbs the viruses or microbes that have not been collected in the dust collection portion. Then, the photocatalyst removes the viruses or microbes adsorbed by the apatite. That is, it is possible to reduce the possibility that light that is projected onto the photocatalyst is blocked by the particulates collected in the dust collection portion when the photocatalyst removes the viruses or microbes adsorbed by the apatite. Accordingly, since light that is sufficient to remove viruses or microbes is projected onto the photocatalyst, the photocatalyst can remove even more viruses or microbes. Consequently, this air purification member can further reduce the generation of unpleasant odor or the occurrence of air contamination.

An air purification member described in claim 12 is the air purification member according to any of claims 1 to 11, further including an antimicrobial portion. The antimicrobial portion inactivates the viruses or inhibits proliferation of the microbes.

This air purification member further includes the antimicrobial portion for inactivating the viruses or inhibiting proliferation of the microbes. Accordingly, even if the microbe removal portion cannot completely remove the viruses or microbes contained in particulates that have been collected by the dust collection portion, the antimicrobial portion can inactivate the viruses or inhibit the proliferation of the microbes. Consequently, this air purification member can further reduce the generation of unpleasant odor or the occurrence of air contamination.

An air purification member described in claim 13 is the air purification member according to claim 12, wherein the antimicrobial portion is carried on the dust collection portion.

Here, the antimicrobial portion is carried on the dust collection portion. Accordingly, the antimicrobial portion carried on the dust collection portion can inactivate the viruses collected in the dust collection portion or inhibit the proliferation of the microbes. Consequently, this air purification member can readily inactivate the collected viruses or inhibit the proliferation of the microbes.

An air purification member described in claim 14 is the air purification member according to claim 12 or 13, wherein the antimicrobial portion includes catechin. Catechin is one kind of polyphenol, and is a generic term for epicatechin, epigallo-catechin, epicatechin gallate and epigallo-catechin gallate, for example.

Here, the antimicrobial portion includes catechin. Catechin is generally known to have an excellent effect of inactivating viruses or inhibiting the proliferation of microbes. Accordingly, it is possible to efficiently inactivate the viruses collected in the dust collection portion or to further inhibit the proliferation of the microbes. Consequently, this air purification member can further reduce the generation of unpleasant odor or the occurrence of air contamination.

An air purification member described in claim 15 is the air purification member according to any of claims 12 to 14, wherein the antimicrobial portion releases a component of Yaku-sugi (Cryptomeriajaponica) bogwood.

Here, the antimicrobial portion releases a component of Yaku-sugi (Cryptomeria japonica) bogwood. Components of Yaku-sugi bogwood are generally known to have an excellent effect of inhibiting the proliferation of microbes. Accordingly, this air purification member can further inhibit the proliferation of microbes. Consequently, this air purification member can further reduce the generation of unpleasant odor or the occurrence of air contamination.

An air purification member described in claim 16 is the air purification member according to any of claims 12 to 15, wherein the antimicrobial portion includes a lytic enzyme.

Here, the antimicrobial portion includes a lytic enzyme. Lytic enzymes are generally known to have an excellent effect of inhibiting the proliferation of microbes since they dissolve the cell walls of microbes. Accordingly, this air purification member can further inhibit the proliferation of microbes.
Consequently, this air purification member can further reduce the generation of unpleasant odor or the occurrence of air contamination.

An air purification member described in claim 17 is the air purification member according to any of claims 1 to 16, wherein the dust collection portion is positively charged.

Viruses, microbes and the like are generally known to have a negative charge. Here, the dust collection portion is positively charged. Accordingly, in this air purification member, the dust collection portion can collect viruses or microbes more efficiently.

An air purification member described in claim 18 is the air purification member according to claim 3, wherein the dust collection portion is an electrode.

Here, the dust collection portion is an electrode. Further, the dust collection portion includes a photocatalyst. Accordingly, viruses or microbes that are adsorbed by the electrode are decomposed by the photocatalyst. Consequently, this air purification member can improve the cleaning efficiency of the electrode.

An air conditioning apparatus described in claim 19 is an air conditioning apparatus for supplying conditioned air into a room, including: a casing; a blowing portion; and an air purification member. The blowing portion blows air that is drawn into the casing into the room. The air drawn into the casing passes through the air purification member. Additionally, this air purification member is the air purification member according to any of claims 1 to 18.

In this air conditioning apparatus, indoor air is drawn into the casing by the blowing portion. The air drawn into the casing passes through the air purification member. At this time, in the air purification member, particulates that are contained in the air are collected by the dust collection portion. Then, viruses or microbes that are contained in the particulates collected by the dust collection portion are removed by the microbe removal portion. Then, purified air is discharged into the room by the blowing portion.

In the case of conventional air conditioning apparatuses, the collected viruses or microbes are left on the air purification member for a long period of time. However, in the above-mentioned air conditioning apparatus, viruses or microbes that are collected by the air purification member are removed by the microbe removal portion. Accordingly, this air conditioning apparatus can reduce the generation of unpleasant odor or the occurrence of air contamination without the need of frequent cleaning or replacement.

An air conditioning apparatus described in claim 20 is the air conditioning apparatus according to claim 19, further including a dehumidification portion. The dehumidification portion dehumidifies the air. Additionally, the air purification member is disposed downstream of the dehumidification portion with respect to a direction of air flow. It should be noted that "dehumidification portion" as mentioned herein refers to a dehumidifier using, for example, a heat exchanger and an adsorbent such as zeolite.

Here, the dehumidification portion dehumidifies the air. Further, the air purification member is disposed downstream of the dehumidification portion in the direction of air flow. Usually, in an air conditioning apparatus, relative humidity tends to be low on the downstream side of the heat exchanger, regardless of whether the air conditioning apparatus is used for heating or for cooling. Basically, viruses, including SARS, tend to favor low-humidity environments. Accordingly, this air conditioning apparatus can efficiently collect viruses. Furthermore, when the air purification member contains an adsorbent such as apatite or zeolite, the apatite or the zeolite tends to adsorb water, rather than viruses, in high-humidity environments. Therefore, the efficiency of collecting viruses can be higher on the downstream side of the heat exchanger, which is relatively dry. Usually, for example, in department stores, air conditioning apparatuses may be used for cooling even in winter, since a large amount of heat is generated in the room. Therefore, the present invention is not limited to the cases where the air conditioning apparatus is used for heating.

An air purification unit described in claim 21 includes a charging portion and an air purification portion. The charging portion charges viruses or microbes that are contained in air. The air purification portion includes apatite. Additionally, this apatite adsorbs the viruses or the microbes. It should be noted that "charging portion" as mentioned herein refers to a plasma ionizer, for example.

Conventionally, there is an air purification member as disclosed in JP H5-068820A. This air purification member carries thereon apatite having adsorption ability, so that it actively adsorbs and collects viruses or microbes passing therethrough. The reason why apatite has excellent adsorption ability with respect to viruses or microbes in this way seems to be that apatite carries an electric charge and thus has the ability to form hydrogen bonds or ionic bonds with viruses or microbes, which have a weak electric charge.

However, in order to efficiently collect viruses or microbes by such apatite, it is required to improve the contact efficiency between apatite and viruses or the like. For example, it is necessary to perform a treatment to render the meshes of the air purification member significantly small. However, rendering the meshes of the air purification member small may hinder the air flow, possibly decreasing the air purification efficiency per unit time.

It is an object of the present invention to provide an air purification unit that can improve the capacity to collect viruses or microbes, without decreasing the air purification efficiency per unit time.

Here, the charging portion charges viruses or microbes that are contained in the air. Furthermore, the air purification portion includes apatite. Accordingly, if this air purification unit can be installed in such a manner that the charging portion is located upstream in the direction of air flow and the apatite is located downstream in the direction of air flow, then more strongly charged viruses or microbes can be drawn to the apatite by an electrostatic effect. Consequently, this air purification unit can improve the capacity to collect viruses or microbes, without decreasing the air purification efficiency per unit time.

An air purification unit described in claim 22 is the air purification unit according to claim 21, wherein the air purification portion further includes a microbe removal portion. The microbe removal portion removes the viruses or the microbes.

Here, the air purification portion further includes a microbe removal portion. Accordingly, in this air purification unit, viruses or microbes that are adsorbed by the apatite are removed by the microbe removal portion. Consequently, this air purification unit can reduce the generation of unpleasant odor or the occurrence of air contamination without the need of frequent cleaning or replacement.

An air purification unit described in claim 23 is the air purification unit according to claim 22, wherein the microbe removal portion includes a photocatalyst.

Here, the microbe removal portion includes a photocatalyst. Accordingly, when light of an appropriate wavelength region is radiated to this photocatalyst, the photocatalyst removes the viruses or microbes collected in the dust collection portion. Consequently, this air purification unit can readily remove viruses or microbes.

An air purification unit described in claim 24 is the air purification unit according to any of claims 21 to 23, wherein the charging portion produces ultraviolet radiation by causing a discharge. It should be noted that "discharge" as mentioned herein refers to a plasma discharge or a corona discharge, for example.

Here, the charging portion produces ultraviolet radiation by causing a discharge. Accordingly, the photocatalyst is activated by the ultraviolet radiation generated by this discharge. It is therefore not necessary to dispose a special light source in the air purification unit. Consequently, it is possible to save the cost required for the light source.

An air purification unit described in claim 25 is the air purification unit according to claim 23 or 24, wherein the air purification portion is an electrode.

Here, the air purification portion is an electrode. Further, the air purification portion includes a photocatalyst. Accordingly, viruses or microbes that are adsorbed by the electrode are decomposed by the photocatalyst. Consequently, this air purification unit can improve the cleaning efficiency of the electrode.

An air conditioning apparatus described in claim 26 is an air conditioning apparatus for supplying conditioned air into a room, including: a casing; a blowing portion; a charging portion; and an air purification portion. The blowing portion blows air that is drawn into the casing into the room. The charging portion charges viruses or microbes that are contained in the air. The air purification portion is provided downstream of the charging portion with respect to a direction of air flow. Further, this air purification portion includes apatite and a microbe removal portion. The apatite adsorbs the viruses or the microbes. The microbe removal portion removes the viruses or the microbes.

In view of its chemical structure, apatite is known to carry an electric charge and to have the ability to form hydrogen bonds or ionic bonds with other substances. Further, since viruses or microbes are constituted by sugar chains, proteins and the like, they have a weak electric charge. The reason why apatite has high adsorption ability with respect to viruses or microbes seems to be that a charge effect acts between them.

Here, the air purification portion is provided downstream of the charging portion in the direction of air flow. Further, this air purification portion includes apatite. Therefore, viruses or microbes are given a stronger electric charge in the charging portion, before they are collected by the air purification portion. Accordingly, viruses or microbes are more easily adsorbed by the apatite. As a result, it is possible to improve the efficiency of collecting viruses or microbes. Further, this air purification portion includes a microbe removal portion. Therefore, viruses or microbes that are adsorbed by the apatite in the air purification portion are removed by the microbe removal portion. Accordingly, this air conditioning apparatus can reduce the generation of unpleasant odor or the occurrence of air contamination without the need of frequent cleaning or replacement.

An air conditioning apparatus described in claim 27 is the air conditioning apparatus according to claim 26, further including a dehumidification portion. The dehumidification portion dehumidifies the air. Further, the air purification member is disposed downstream of the dehumidification portion in the direction of air flow. It should be noted that "dehumidification portion" as mentioned herein refers to a dehumidifier using, for example, a heat exchanger and an adsorbent such as zeolite.

Here, the dehumidification portion dehumidifies the air. Further, the air purification member is disposed downstream of the dehumidification portion in the direction of air flow. Usually, in an air conditioning apparatus, relative humidity tends to be low on the downstream side of the heat exchanger, regardless of whether the air conditioning apparatus is used for heating or for cooling. Basically, viruses, including SARS, tend to favor low-humidity environments. Accordingly, this air conditioning apparatus can efficiently collect viruses. Furthermore, when the air purification member contains an adsorbent such as apatite or zeolite, the apatite or the zeolite tends to adsorb water, rather than viruses, in high-humidity environments. Therefore, the efficiency of collecting viruses can be higher on the downstream side of the heat exchanger, which is relatively dry. Usually, for example, in department stores, air conditioning apparatuses may be used for cooling even in winter, since a large amount of heat is generated in the room. Therefore, the present invention is not limited to the cases where the air conditioning apparatus is used for heating.

An air conditioning apparatus described in claim 28 is the air conditioning apparatus according to claim 26 or 27, wherein the microbe removal portion is a photocatalyst.

Here, the microbe removal portion is a photocatalyst. Accordingly, when light of an appropriate wavelength region is radiated to this photocatalyst, the photocatalyst removes the viruses or microbes that are collected in the dust collection portion. Consequently, this air conditioning apparatus can readily remove viruses or microbes.

An air conditioning apparatus described in claim 29 is the air conditioning apparatus according to claim 28, wherein the apatite and the photocatalyst are the same substance.

That is to say, this substance is a photocatalytic apatite. A photocatalytic apatite is, for example, a substance in which a portion of the calcium atoms constituting calcium hydroxyapatite apatite has been replaced by titanium atoms, and has both photocatalytic activity and adsorption ability, which is unique to apatite.

Conventionally, for example, a mixture of an adsorbent, such as zeolite, and titanium oxide is used as a photocatalyst, for example. On the other hand, apatite is known to have a high adsorption property with respect to viruses or microbes, and it is conceivable to adopt a configuration in which apatite is used in place of the adsorbent in the above-described mixture, in order to increase the effect of the microbe removal portion. However, even if adopted, such a configuration is only effective for viruses or microbes that are adsorbed in the vicinity of titanium oxide, which exhibits catalytic activity. When viruses or microbes are adsorbed in apatite, without the presence of titanium oxide in the vicinity thereof, they cannot be removed and thus remain on the apatite, thus possibly causing the generation of unpleasant odor or air contamination after an elapse of a long period. However, since the above-mentioned apatite having photocatalytic activity has photocatalytic activity in its adsorption site, it is possible to remove the adsorbed viruses or microbes substantially completely. Consequently, this air conditioning apparatus can reduce the generation of unpleasant odor or the occurrence of air contamination without necessitating frequent cleaning or replacement of the air purification member.

An air conditioning apparatus described in claim 30 is the air conditioning apparatus according to claim 28 or 29, wherein the charging portion produces ultraviolet radiation by causing a discharge.

Here, the charging portion produces ultraviolet radiation by causing a discharge. Accordingly, the photocatalyst is activated by the ultraviolet radiation produced by this discharge. It is therefore not necessary to dispose a special light source in this air conditioning apparatus. Consequently, it is possible to save the cost required for the light source.

An air conditioning apparatus described in claim 31 is the air conditioning apparatus according to any of claims 28 to 30, wherein the air purification portion is an electrode.

Here, the air purification portion is an electrode. Further, the air purification portion includes a photocatalyst. Accordingly, viruses or microbes that are adsorbed by the electrode are decomposed by the photocatalyst. Consequently, this air purification member can improve the cleaning efficiency of the electrode.

An air purification member described in claim 32 is an air purification member for purifying air that contains particulates containing viruses or microbes and that has a suspended dust concentration of 0.15 mg/m$^3$ or less, in-

cluding: a HEPA filter; and apatite. The apatite is provided in the HEPA filter and adsorbs the viruses or the microbes. The method for calculating "suspended dust concentration" is well known to the person skilled in the art. Additionally, "HEPA" as mentioned herein is an acronym for High Efficiency Particulate Air Filter, and a generic term for filters having the performance to remove 99.97% or more of all kinds of particulates having a size of 0.3 μm or larger (the global standard, i.e., the NASA standard), whether they are dust, pollen or bacteria. Further, in the present specification, HEPA filters include ULPA filters. "ULPA" as mentioned herein is an acronym for an Ultra low penetration air, and a generic term for filters having the performance to remove 99.995% or more of all kinds of particulates having a size of 0.1 μm or larger, whether they are dust, pollen or bacteria.

Conventionally, the HEPA filter disclosed in Non-patent document 1 (Uichi Inoue ed., "Kuki Chowa Handbook (Air conditioning handbook)". 4th ed, Maruzen Co. Ltd., Apr. 2002, p. 277) is often used for the operating rooms or sterile rooms in hospitals, sterile tents for asthma patients, biotechnology laboratories, semiconductor plants and other places where clean air is required. This is because HEPA filters have excellent dust collection ability, as described above.

However, some viruses or microbes have a size of 0.3 μm or smaller, and, therefore, even a HEPA filter cannot guarantee complete removal of viruses or microbes.

It is an object of the present invention to provide an air purification member having better ability to collect viruses or microbes than HEPA filters.

Here, apatite is provided in the HEPA filter to adsorb viruses or microbes. Apatite is generally known to exhibit excellent adsorption ability with respect to viruses or microbes. Accordingly, this air purification member has better ability to collect viruses or microbes than HEPA filters.

An air purification member described in claim 33 is the air purification member according to claim 32, wherein the apatite is provided on an upstream surface of the HEPA filter with respect to a direction of air flow.

Here, the apatite is provided on the upstream surface of the HEPA filter in the direction of air flow.

In general, viruses or microbes tend to adhere to dust or dirt particles. Therefore, even viruses or microbes having a size of 0.3 μm or smaller can be usually blocked on the upstream surface of the dust collection portion in the direction of air flow, as long as they are adhering to dust or dirt particles. Accordingly, more viruses or microbes are supposed to be present on the upstream surface of the dust collection portion in the direction of air flow. Here, the apatite is provided on the upstream surface of the dust collection portion in the direction of air flow. Consequently, viruses or microbes can be efficiently adsorbed by the apatite.

An air purification member described in claim 34 is the air purification member according to claim 33, further including a photocatalyst. This photocatalyst is provided on the upstream surface of the HEPA filter in the direction of air flow.

Here, the photocatalyst is provided on the upstream surface of the HEPA filter in the direction of air flow. Accordingly, when light of an appropriate wavelength region is radiated to this photocatalyst, the photocatalyst removes viruses or microbes adsorbed by the apatite. Consequently, this air purification member can readily remove viruses or microbes.

An air purification member described in claim 35 is the air purification member according to claim 34, wherein the apatite and the photocatalyst are apatite having photocatalytic activity, or a mixed photocatalyst. Additionally, this mixed photocatalyst is a mixture of apatite having photocatalytic activity and a photocatalytic material.

Here, the apatite and the photocatalyst are apatite having photocatalytic activity or a mixed photocatalyst. Accordingly, this air purification member can remove viruses or microbes with higher efficiency.

An air purification unit described in claim 36 includes: an air purification member; and a light source. The air purification member is the air purification member according to claim 34 or 35. The light source is disposed in a space located upstream of the HEPA filter with respect to a direction of air flow.

In general, viruses or microbes tend to adhere to dust or dirt particles. Therefore, even viruses or microbes having a size of 0.3 μm or smaller can be usually blocked on the upstream surface of the dust collection portion in the direction of air flow, as long as they are adhering to dust or dirt particles. Accordingly, more viruses or microbes are supposed to be present on the upstream surface of the dust collection portion in the direction of air flow. Here, the air purification member is the air purification member according to claim 30 or 31. Further, the light source is disposed in a space located upstream of the HEPA filter in the direction of air flow. Accordingly, this air purification unit can remove viruses or microbes with higher efficiency.

**Brief Description of Drawings**

FIG. 1 is an external perspective view of an air purifier according to an embodiment of the present invention.
FIG. 2 is an exploded perspective view of filters and a blowing mechanism according to an embodiment of the present invention.

FIG. 3 is a block diagram schematically showing a control portion according to an embodiment of the present invention.

FIG. 4 is a detailed view of a pre-filter according to an embodiment the present invention.

FIG. 5 is an enlarged cross-sectional view of the net portion of a pre-filter according to an embodiment of the present invention.

FIG. 6 is a diagram showing a partial cross-sectional view of a roll filter according to an embodiment of the present invention.

FIG. 7 is an enlarged cross-sectional view of the roll filter when the roll filter collects particulates.

FIG. 8 is an external perspective view of an air conditioner according to another embodiment of the present invention.

FIG. 9 is a diagram showing the system configuration of the air conditioning system according to an embodiment of the present invention.

FIG. 10 is a detailed view of an advanced HEPA filter according to an embodiment of the present invention.

FIG. 11 is a diagram showing the system configuration of an air conditioning system according to an embodiment the present invention.

**Best Mode for Carrying Out the Invention**

Hereinafter, preferred embodiments of the present invention will be described in detail with reference to the accompanying drawings.

*First embodiment*

<Overall configuration of air purifier>

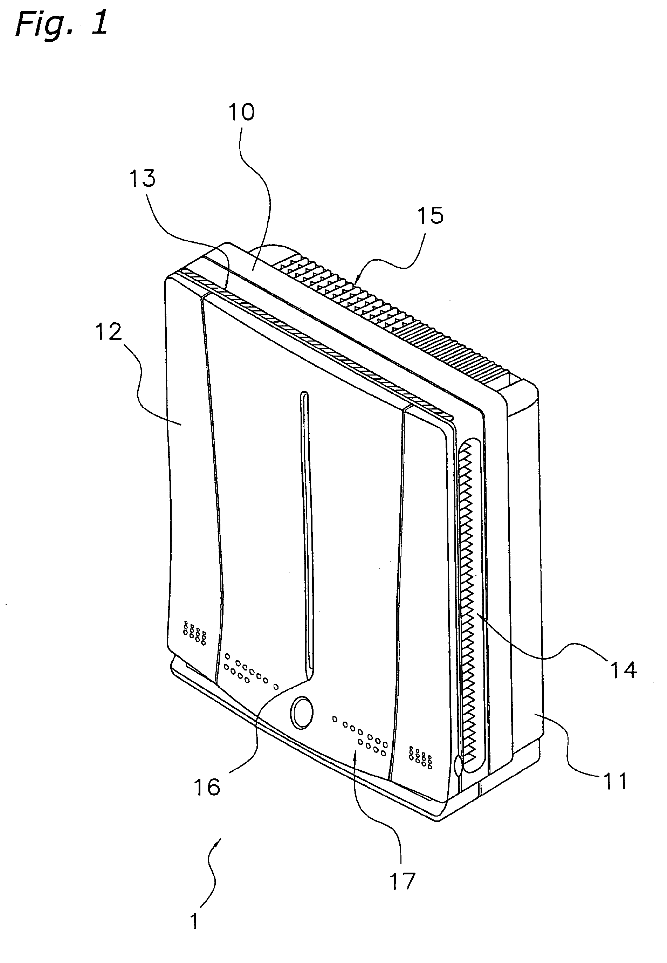

FIG. 1 shows an external view of an air purifier 1 for which one embodiment of the present invention is employed.

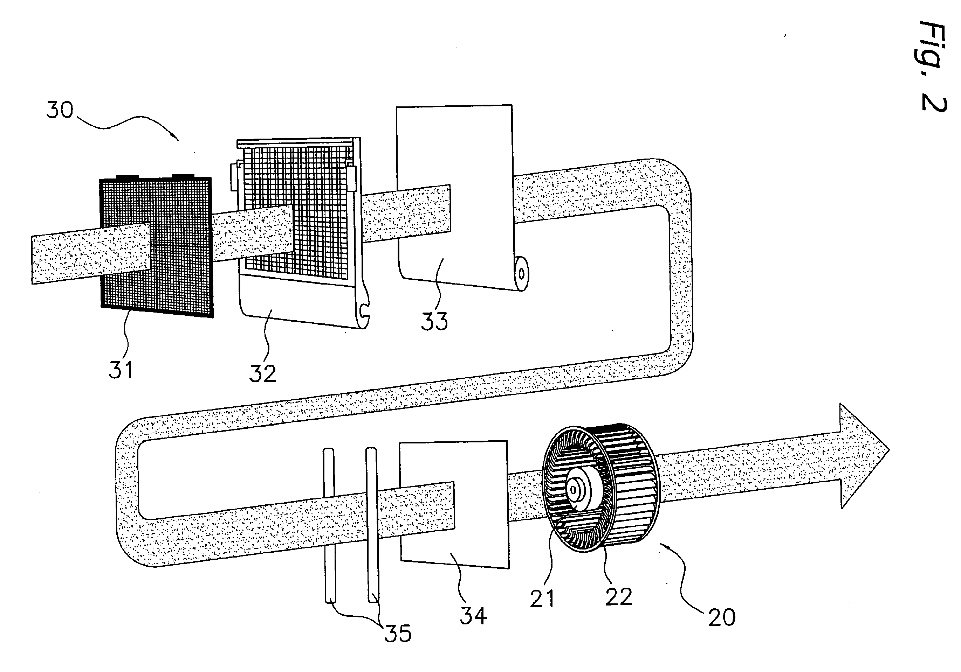

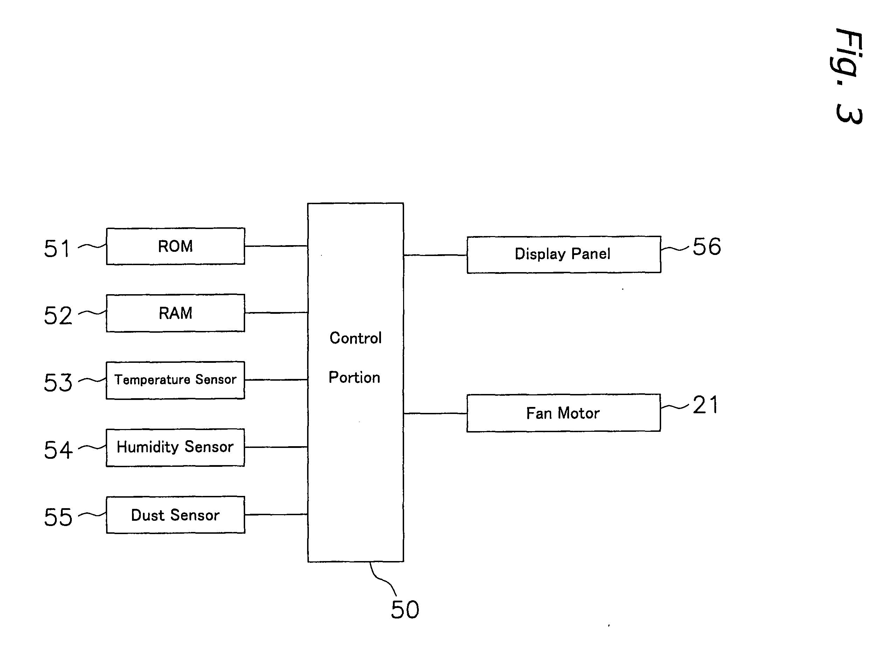

The air purifier 1 maintains a comfortable indoor environment by purifying the air in a room in a building or a residence and blowing the purified air into the room. The air purifier 1 is provided with a casing 10, a blowing mechanism 20 (see FIG. 2), a control portion 50 (see FIG. 3) and a filter unit 30 (see FIG. 2).

The casing 10 constitutes the external surface of the air purifier 1, and contains the blowing mechanism 20, the control portion 50 and the filter unit 30. The casing 10 has a body portion 11 and a front panel 12.

The body portion 11 has an upper surface intake port 13, side surface intake ports 14 and a discharge port 15. The upper surface intake port 13 and the side surface intake ports 14 are substantially rectangular openings for drawing indoor air into the air purifier 1, in order to purify the indoor air in the air purifier 1. The upper surface intake port 13 is provided at the front-side end on the upper side of the body portion 11, which is also the side on which the discharge port 15 is provided. The side surface intake ports 14 are a pair of openings that are provided on the right-side surface and the left-side surface, respectively, of the body portion 11. The discharge port 15 is provided at the rear end on the upper side of the body portion 11. The discharge port 15 is an opening for discharging purified air from the air purifier 1 into the room.

The front panel 12 is provided at the front of the body portion 11, and covers the filter unit 30 that is placed inside the body portion 11. The front panel 12 has a front intake port 16 and a display panel opening 17. The front intake port 16 is a substantially rectangular opening that is provided at substantially the center of the front panel 12 for drawing indoor air into the air purifier 1. The display panel opening 17 is provided such that a display panel 56, which will be described later, can be viewed from the outside of the casing 10.

The blowing mechanism 20 draws in indoor air from the intake ports (the upper surface intake port 13, the side surface intake ports 14 and the front intake port 16), and discharges purified air from the discharge port 15. The blowing mechanism 20 is provided inside the casing 10, and is configured so as to allow indoor air that is drawn in from the intake ports to pass through the filter unit 30. Furthermore, as shown in FIG. 2, the blowing mechanism 20 is provided with a fan motor 21 and a blowing fan 22 that is rotationally driven by the fan motor 21. For the fan motor 21, an inverter motor whose frequency is controlled by an inverter circuit may be used. For the blowing fan 22, a centrifugal fan may be used.

The air purifier 1 further includes a control portion 50 that is constituted by a microprocessor. As shown in FIG. 3, the control portion 50 is connected to, for example, a ROM 51 for storing a control program, various parameters and the like, and a RAM 52 for temporarily storing currently processed variables and the like.

The control portion 50 is also connected to various sensors, such as a temperature sensor 53, a humidity sensor 54 and a dust sensor 55, and receives input of the detection signals of the sensors. The dust sensor 55 can measure the concentration of particles such as dust by radiating light into supplied air, and detecting the amount of light that has reached the light-receiving element after being scattered by smoke, dust, pollen and other particles that are contained in the air.

Further, the control portion 50 is connected to the display panel 56. The display panel 56 displays, for example, the operation mode, monitoring information by the sensors, timer information and maintenance information, and can be viewed by the user and the like from the outside through the display panel opening 17. The display panel 56 can also be constituted by a liquid crystal display panel, an LED, any other display device, or a combination of these devices.

Furthermore, the control portion 50 is connected to the fan motor 21, and can control the operation of such devices in accordance with the user operation or the detection results obtained by the sensors.

<Configuration of filter unit>

The filter unit 30 is provided inside the casing 10, and removes particulates that are contained in the indoor air drawn in from the intake ports 13, 14 and 16. As shown in FIG. 2, the filter unit 30 has a pre-filter 31, a plasma ionization portion 32, a first photocatalytic filter 33, a second photocatalytic filter 34 and inverter lamps 35. The filter unit 30 is configured in such a manner that the indoor air drawn in from the intake ports passes through the filter unit 30 in this order: the pre-filter 31, the plasma ionization portion 32, the first photocatalytic filter 33 and the second photocatalytic filter 34.

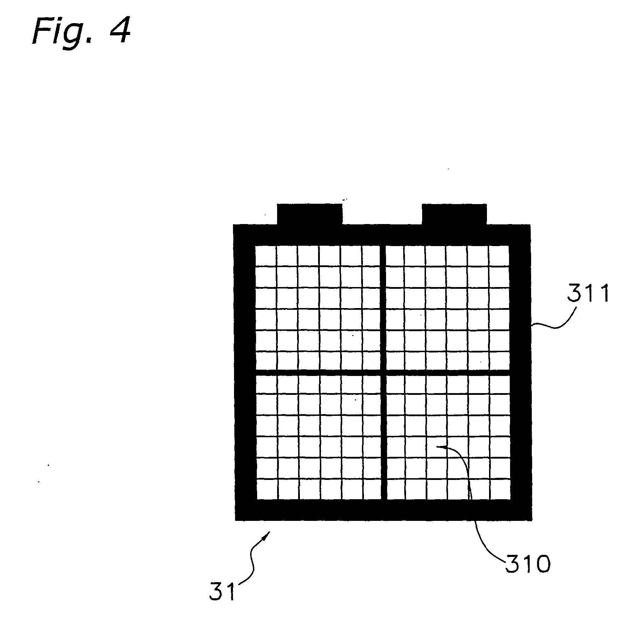

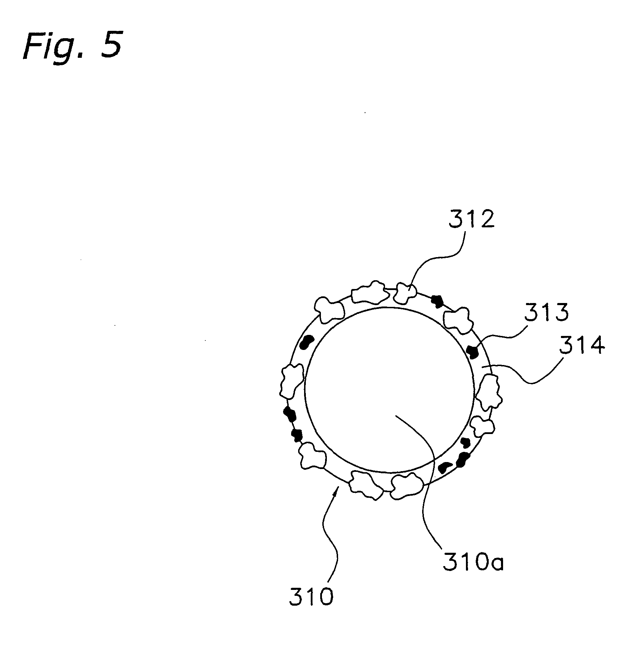

The pre-filter 31 is a filter for removing relatively large dust or the like from air that is drawn into the casing 10 by the blowing mechanism 20. The pre-filter 31 has a net portion 310 and a frame 311 (see FIG. 4). The net portion 310 is a resin net made of polypropylene (hereinafter, referred to as "PP") filaments, to which relatively large dust particles or the like that are contained in the air drawn into the casing 10 adhere. In addition, the fiber constituting the net portion 310 is made up of a core 310a made of PP and a covering layer 314 also made of PP. A visible light photocatalyst 312 and catechin 313 are carried on the covering layer 314 such that they are exposed to the air side (see FIG. 5). The visible light photocatalyst 312 contains, for example, titanium oxide, whose photocatalytic effect is activated by visible light, and removes microbes, such as fungi and bacteria, or viruses that are contained in dust or the like adhering to the net portion 310. Catechin is one kind of polyphenol, and is a generic term for epicatechin, epigallo-catechin, epicatechin gallate and epigallo-catechin gallate, for example. This catechin suppresses the proliferation of microbes such as fungi and bacteria that are contained in dust or the like adhering to the net portion 310, and inactivates viruses (see FIG. 5).

The plasma ionization portion 32 charges dust or the like contained in the air that has passed through the pre-filter 31 by applying a strong electric charge thereto. By this charging, the efficiency of collecting dust or the like in an electrostatic filter 330, which will be described later, of the first photocatalytic filter 33 can be improved. Furthermore, the plasma ionization portion 32 also charges viruses or microbes that are contained in the dust, so that it is possible to improve the efficiency of adsorbing viruses or microbes in a photocatalytic apatite, which will be described later, thus improving the efficiency of removing viruses or microbes.

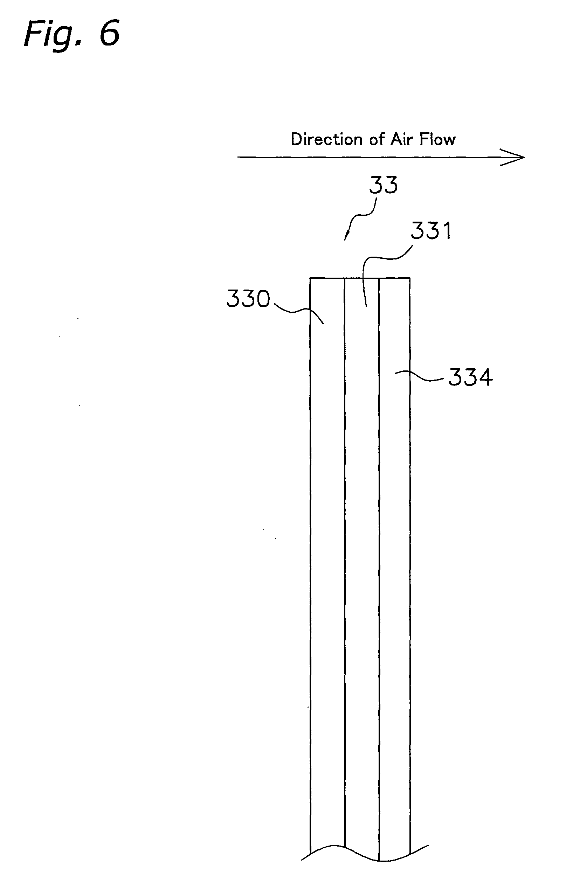

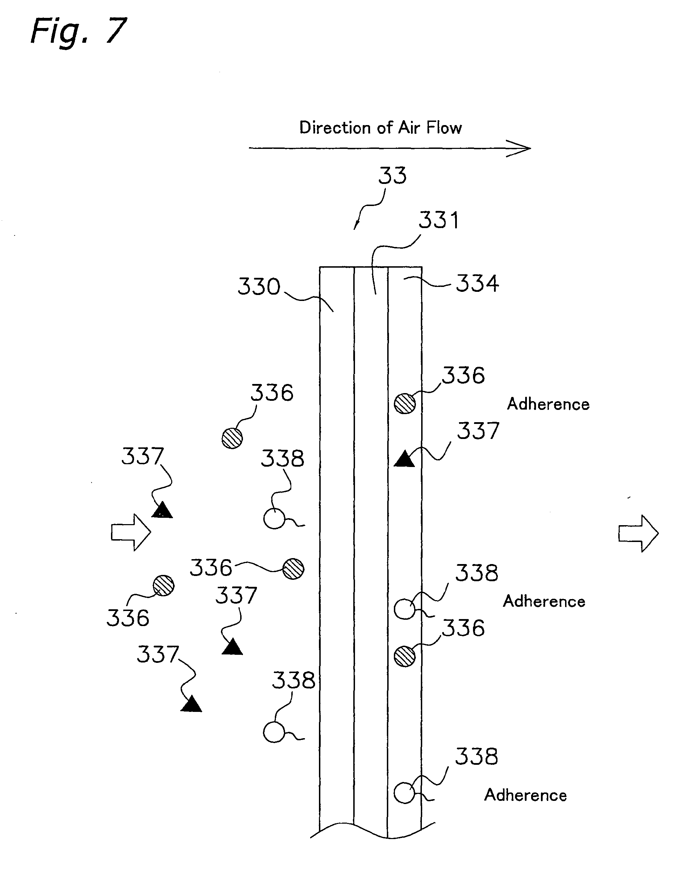

FIG. 6 shows a partial cross-sectional view of the first photocatalytic filter 33. The first photocatalytic filter 33 is formed into the shape of a roll that is made by winding a filter having a length of a plurality of turns, and is configured such that when the used filter area is soiled, then it can be pulled out and the soiled portion can be cut off. The first photocatalytic filter 33 has an electrostatic filter 330 and a photocatalytic filter 331. The electrostatic filter 330 and the photocatalytic filter 331 are laminated to form the first photocatalytic filter 33. The electrostatic filter 330 and the photocatalytic filter 331 are disposed upstream and downstream, respectively, in the air flow generated by the blowing mechanism 20. The electrostatic filter 330 adsorbs dust or the like that has been charged in the plasma ionization portion 32. The dust or the like passing through electrostatic filter 330 adheres to the photocatalytic filter 331. Photocatalytic apatite 334, in which the calcium atoms of calcium hydroxyapatite have been replaced by titanium atoms, is carried on the surface of the photocatalytic filter 331 that is located downstream with respect to the air flow. The photocatalytic apatite 334 removes, for example, viruses, fungi or bacteria contained in dust or the like by adsorption. Furthermore, the photocatalytic apatite 334 also adsorbs and decomposes ammonias, aldehydes 336 and nitrogen oxides 337 that are contained in the air passing through the photocatalytic filter 331 (see FIG. 7). Table 1 shows the inactivation rate of viruses, microbes and toxins by the photocatalytic apatite 334. As is evident from Table 1, the photocatalytic apatite 334 shows an inactivation rate of 99.99% or higher for an influenza virus, Escherichia coli (O-157), Staphylococcus aureus and Cladosporium cladosporioides. Moreover, the photocatalytic apatite 334 also shows an inactivation rate of 99.9% or higher for an enterotoxin (toxin).

Titanium oxide, which has an photocatalytic effect, is carried on the second photocatalytic filter 34. The second photocatalytic filter 34 adsorbs dirt or dust contained in the air that has not been adsorbed by the first photocatalytic filter 33. The second photocatalytic filter 34 removes fungi, bacteria or viruses contained in the adsorbed dirt or dust using the titanium oxide.

The inverter lamps 35 are disposed between the first photocatalytic filter 33 and the second photocatalytic

**EP 1 600 201 A1**

filter 34. The inverter lamps 35 radiate ultraviolet radiation to the photocatalytic filter 331 of the first photocatalytic filter 33 and the second photocatalytic filter 34, and activate the photocatalytic effect of the photocatalytic filters.

Table 1

| test subject | | inactivation rate | testing laboratory/certification No. |
|---|---|---|---|
| influenza virus | | 99.99% or higher | Japan Food Research Laboratories No. 203052102 |
| microbes | Escherichia coli (O-157) | 99.99% or higher | Japan Food Research Laboratories No. 203030567-001 |
| | Staphylococcus aureus | 99.99% or higher | Japan Food Research Laboratories No. 203030567-001 |
| | Cladosporium cladosporioides | 99.99% or higher | Japan Food Research Laboratories No. 203030567-001 |
| toxin | enterotoxin | 99.9% or higher | Japan Food Research Laboratories No. 203050715-001 |

It should be noted that these inactivation rates were measured at Japan Food Research Laboratories, using the following methods.

<Inactivation rate of influenza virus>

(1) Test overview

An influenza virus suspension was dropped onto a filter (approx. 30 mm $\times$ 30 mm) on which the photocatalytic apatite 334 was applied, and the filter was stored at room temperature under dark conditions (shade) and light conditions [under black light irradiation (the distance between the filter and the black light: approx. 20 cm)]. Twenty four hours later, the virus infectivity titer was determined.

(2) Calculation of inactivation rate

$$\text{inactivation rate} = 100 \times (1 - 10^B/10^A)$$

A: virus infectivity titer immediately after inoculation
B: virus infectivity titer of filter after 24 hours of light irradiation

(3) Test method

A. Test virus: influenza type A virus (H1N1)
B. Cells used: MDCK (NBL-2) cell ATCC CCL-34 (Dainippon Pharmaceutical Co., Ltd.)
C. Culture medium used

a) Growth medium

Eagle MEM (0.06 mg/ml, containing kanamycin) to which 10% newborn bovine serum was added was used.

b) Maintenance medium

A culture medium having the following composition was used.

| Eagle MEM | 1,000 mL |
|---|---|
| 10% NaHCO3 | 24 to 44 mL |
| L-glutamine (30 g/L) | 9.8 mL |
| $100 \times$ MEM vitamin solution | 30 mL |
| 10% albumin | 20 mL |
| trypsin (5 mg/mL) | 2 mL |

D. Preparation of virus suspension

a) Cell culture

Using the growth medium, the MDCK cells were cultured in a monolayer in a tissue culture flask.

b) Inoculation of virus

After the cells were cultured in a monolayer, the growth medium was removed from the flask, and the test virus was inoculated. Then, the maintenance medium was added, and culture was performed in a carbon dioxide incubator ($CO_2$ concentration: 5%) at 37°C for 2 to 5 days.

c) Preparation of virus suspension

After culture, the morphology of the cells was examined with an inverted phase contrast microscope, and it was confirmed that 80% or more of the cells underwent a morphological change (cell denaturation effect). Then, the culture solution was subjected to centrifugal separation (3,000 r/min, 10 min), and the resulting supernatant fluid was used as the virus suspension.

E. Preparation of sample

The filter (approx. 30 mm $\times$ 30 mm) was subjected to moist heat sterilization (121°C, 15 min), and thereafter air-dried for one hour. The filter was placed in a plastic petri dish, and then irradiated with black light (black light blue, FL20S BL-B 20 W, two tubes in parallel) for at least 12 hours. This was used as a sample.

F. Test procedure

0.2 mL of the virus suspension was dropped onto the sample. The sample was stored at room temperature under shade and under irradiation of black light (distance between the filter and the black light: approx. 20 cm). In addition, as a control sample, a polyethylene film was tested in the same manner.

G. Washing of the Virus

After storage for 24 hours, the virus suspension in the test strip was washed out with 2 mL of the maintenance medium.

H. Determination of virus infectivity titer

Using the growth medium, the MDCK cells were cultured in a monolayer in a tissue culture microplate (96 wells), and thereafter the growth medium was removed, followed by adding 0.1 mL each of the maintenance medium. Then, 0.1 mL each of the washed solution and its diluent were inoculated into four wells each, and culture was performed in a carbon dioxide incubator ($CO_2$ concentration: 5%) at 37°C for 4 to 7 days. After culture, the presence or absence of any morphological change of the cells (cell denaturation effect) was examined with an inverted phase contrast microscope. Then, the 50 percent tissue culture infectious dose ($TCID_{50}$) was calculated by the Reed-Muench method, and this was converted into the virus infectivity titer per mL of the washed solution.

<Inactivation rate of Escherichia coli (O-157), Staphylococcus aureus and Cladosporium cladosporioides>

(1) Test overview

The antimicrobial efficacy of the filter was tested with reference to a testing method of the Society of Industrial-Technology for Antimicrobial Articles (SIAA), namely "the method for evaluating the antimicrobial efficacy of antimicrobial products III (2001): Film contact method on optical irradiation" (hereinafter, referred to as "film contact method on optical irradiation (SIAA, 2001)").
The testing was performed as follows:
Each of the liquids of Escherichia coli, Staphylococcus aureus and Cladosporium cladosporioides was dropped onto the sample, and a low-density polyethylene film was placed over the sample so as to adhere to the samples. These samples were stored at room temperature (20 to 25°C), under dark conditions (shade) and light conditions [under irradiation of black light (distance between the filter and the black light: approx. 20 cm)], and the viable count after 24 hours was measured.

(2) Testing method

A. Test strains

Bacteria:

Escherichia coli IFO 3972
Staphylococcus aureus subsp. aureus IFO 12732

Fungus:

Cladosporium cladosporioides IFO 6348

B. Test media

NA medium: a nutrient agar medium (EIKEN CHEMICAL CO., LTD.)
1/500 NB medium: a medium prepared by diluting an ordinary broth (EIKEN CHEMICAL CO., LTD.) to which 0.2% meat extract was added by a phosphate buffer solution to 500-fold and adjusting the pH to 7.0±0.2
SCDLP medium: a SCDLP medium (NIHON PHARMACEUTICAL CO., LTD)
SA medium: a standard agar medium (EIKEN KIZAI CO., LTD)
PDA medium: a potato dextrose agar medium (EIKEN KIZAI CO., LTD)
C. Preparation of microbial liquids

Bacteria:

Each of the test strains that had been pre-cultured in the NA medium at 35°C for 16 to 24 hours was re-inoculated into the NA medium, and the bacteria cells that had been cultured at 35°C for 16 to 20 hours were uniformly dispersed in the 1/500 NB medium. Then, each of the bacteria liquids was prepared such that the number of bacteria per mL was $2.5 \times 10^5$ to $1.0 \times 10^6$.

Fungus:

After culture in the PDA medium at 25°C for 7 to 10 days, the spores (conidiums) were suspended in a 0.005% dioctyl sodium sulfosuccinate solution, and filtered with gauze. Then, the fungi liquid was prepared such that the number of spores per mL was $2.5 \times 10^5$ to $1.0 \times 10^6$.

D. Preparation of samples

The filter (approx. 50 mm $\times$ 50 mm) was subjected to moist heat sterilization (121°C, 15 min), and thereafter air-dried for one hour. The filter was placed in a plastic petri dish, and then irradiated with black light (black light blue, FL20S BL-B 20 W, two tubes in parallel) for at least 12 hours. This filter was used as a sample.

E. Test procedure

0.4 mL of each of the microbial liquids was dropped onto the sample, and a low-density polyethylene film (40 mm × 40 mm) was placed over the sample so as to adhere to the sample. These samples were stored at room temperature (20 to 25°C), under shade and under irradiation of black light (the distance between the filter and the black light: approx. 20 cm). In addition, as a control sample, a polyethylene film was tested in the same manner.

F. Measurement of viable count

After storage for 24 hours, any living bacteria were washed out from the samples with the SCDLP medium, and the viable count in this washed solution was measured by a poured plate method, using the SA medium (35°C, 2-day culture) for the bacteria, and the PDA medium (25°C, 8-day culture) for the fungus, and the measurement results were each converted into a value per sample. In addition, the measurement was carried out immediately after inoculation for the control sample.

<Inactivation rate of enterotoxin>

(1) Test overview

Staphylococcus enterotoxin A (hereinafter, abbreviated as "SET-A") was inoculated into the sample, and stored at room temperature (20 to 25°C) under dark conditions (shade) and light conditions (under light irradiation of ultraviolet radiation with an intensity of about 1 mW/cm$^2$). Twenty four hours later, the SET-A concentration was measured and the decomposition rate was calculated.

(2) Test method

A. Preparation of standard undiluted solution

A standard product of SET-A [TOXIN TECHNOLOGY] was dissolved in a 1% sodium chloride solution containing 0.5% bovine serum albumin, thus preparing a 5 μm/mL standard undiluted solution.

B. Standard solution for calibration curve

The standard undiluted solution was diluted with the buffer solution included with VIDAX Staph enterotoxin (SET) [bioMerieux] to prepare 0.2 ng/mL, 0.5 ng/mL and 1 ng/mL standard solutions.

C. Preparation of sample

The filter was cut into 50 mm × 50 mm, and irradiated with black light from a distance of about 1 cm for 24 hours. This was used as a sample.

D. Test procedure

The sample was placed into a plastic petri dish, and inoculated with 0.4 mL of the SET-A standard undiluted solution. This was stored at room temperature (20 to 25°C) under shade and under light irradiation of ultraviolet radiation with an intensity of about 1 mW/cm$^2$ (black light, FL 20S BL-B 20 W, two tubes in parallel).
After storage of 24 hours, the SET-A was washed out from the sample with 10mL of the buffer solution included with VIDAX·Staph enterotoxin (SET) [bioMerieux]. This was used as a sample solution.
In addition, 0.4 mL of the standard undiluted solution of SET-A was inoculated into a plastic petri dish in which no sample was placed. Immediately thereafter, 10mL of the buffer solution included with VIDAX Staph enterotoxin (SET) (bioMerieux) was added, and this was used as a control.

E. Creation of calibration curve

For the standard solution for calibration curve, a measurement was made by an ELISA method using VIDAX Staph enterotoxin (SET) [bioMerieux], and a calibration curve was created based on the concentration and the fluorescence intensity of the standard solution.

F. Measurement of SET-A concentration and calculation of decomposition rate

The fluorescence intensity of the sample solution was measured by an ELISA method using VIDAX Staph enterotoxin (SET) [bioMerieux]. Then, the SET-A concentration was determined from the calibration curve created as described in section E above, and the decomposition rate was calculated by the following equation.

$$\text{decomposition rate (\%)} = (\text{measured value of control} - \text{measured value of}$$

$$\text{sample solution})/\text{measured value of control} \times 100$$

<Feature of air purifier of this embodiment>

(1)

In the case of conventional air purifiers, viruses, fungi or bacteria that are contained in dust or the like adhering to the net portion of the pre-filter are left adhering to the net portion until the pre-filter is cleaned, for example, by the user of the air purifier. Accordingly, in conventional air purifiers, there is the possibility that viruses may be released again, or fungi or bacteria may proliferate on the net portion, thus causing unpleasant odor or air contamination.

However, in the case of the air purifier 1, relatively large dust or the like contained in indoor air adheres to the net portion 310 of the pre-filter 31. Then, viruses, fungi or bacteria that are contained in the dust or the like adhering to the net portion 310 are removed by the visible light photocatalyst 312. Consequently, the air purifier 1 can reduce the generation of unpleasant odor or the occurrence of air contamination without the need of frequent cleaning of the pre-filter 31.

(2)

In the case of conventional air purifiers, viruses, fungi or bacteria that are contained in dust or the like adhering to the filter are left adhering to the net portion until the filter is replaced, for example, by the user of the air purifier. Accordingly, in conventional air purifiers, there is the possibility that viruses may be released again, or fungi or bacteria may proliferate on the net portion, thus causing unpleasant odor or air contamination.

However, in the case of the air purifier 1, dust or the like that is contained in air adheres to the photocatalytic filter 331. Then, viruses, fungi or bacteria that are contained in the dust or the like adhering to the photocatalytic filter 331 are removed by the photocatalytic apatite 334 by adsorption. Consequently, the air purifier 1 can reduce the generation of unpleasant odor or the occurrence of air contamination without the need of frequent replacement of the first photocatalytic filter 33.

Furthermore, in the air purifier 1, viruses, fungi or bacteria that are contained in the air are removed by the visible light photocatalyst 312 or the photocatalytic apatite 334. Accordingly, the air purifier 1 can readily remove viruses, fungi or bacteria.

(3)

In the air purifier 1, the visible light photocatalyst 312 is carried on the surface of the net portion 310 of the pre-filter 31. Accordingly, when indoor light is projected onto the net portion 310, the photocatalytic activity of the photocatalyst 312 is activated. That is, it is possible to remove viruses, fungi or bacteria without preparing a special light source. Consequently, the air purifier 1 can remove viruses, fungi or bacteria with a simple configuration.

(4)

In the air purifier 1, the net portion 310 of the pre-filter 31 is constituted by a fiber. This fiber is made up of the core 310a and the covering layer 314. Further, the covering layer 314 holds the photocatalyst 312 such that a portion of the photocatalyst 312 is exposed to the air side.

In general, a photocatalyst is carried on fiber using a method such as performing injection molding, with powder or the like of the photocatalyst dispersed in resin. However, such fiber gradually deteriorates each time it is irradiated with light having a wavelength to which that photocatalyst produces catalytic action. Accordingly, a filter that is constituted by such fiber will gradually lose its strength. Furthermore, there is also a problem in that an object that is made of resin containing any foreign substance generally tends to be fragile.

However, the fiber 310 has the core 310a, and the photocatalyst 312 is carried only on the covering layer 314. Accordingly, although the covering layer 314 gradually deteriorates each time it is irradiated with light having a wavelength to which the photocatalyst 312 produces catalytic reaction, the core 310a will not deteriorate owing to the light. Moreover, the core 310a has favorable strength, since no foreign substance (photocatalyst) is mixed into the core. Accordingly, the pre-filter 31 can maintain its strength for a long period of time.

(5)

In the air purifier 1, the photocatalytic apatite 334 is carried on the downstream surface of the photocatalytic filter 331 in the direction of air flow. Conventionally, for example, a mixture of an adsorbent, such as zeolite, and titanium oxide is used as a photocatalytic filter. On the other hand, apatite is known to have high adsorption property with respect to viruses or microbes, and it is conceivable to adopt a configuration in which apatite is used in place of the adsorbent in the above-mentioned photocatalytic filter, in order to increase the effect of the microbe removal portion. However, even if adopted, such a configuration is only effective for viruses or microbes that are adsorbed in the vicinity of titanium oxide, which exhibits catalytic activity. When viruses or microbes are adsorbed in apatite, without the presence of titanium oxide in the vicinity thereof, they cannot be removed and thus remain on the apatite, thus possibly causing the generation of unpleasant odor or air contamination after a long period of time has elapsed. However, since the photocatalytic apatite 334 has photocatalytic activity in its adsorption site, it is possible to remove the adsorbed viruses, microbes or the like substantially completely. Consequently, the photocatalytic filter 331 can reduce the generation of unpleasant odor or the occurrence of air contamination without necessitating frequent cleaning or replacement.

(6)

In the air purifier 1, the first catalytic filter 33 is provided downstream of the plasma ionization portion 32 in the direction of air flow. Further, the photocatalytic apatite 334 is carried on the first catalytic filter 33.

In view of its chemical structure, apatite is known to carry an electric charge and to have the ability to form hydrogen bonds or ionic bonds with other substances. Further, since viruses or microbes are constituted by sugar chains, proteins and the like, they have a weak electric charge. The reason why apatite has high adsorption ability with respect to viruses or microbes seems to be that an influence of the electric charge is exerted between them.

That is, since the air purifier 1 adopts a configuration as described above, viruses or microbes are given a stronger electric charge in the plasma ionization portion 32, before they are collected by the filter. Accordingly, viruses or microbes are more easily adsorbed by the photocatalytic apatite 334. As a result, it is possible to improve the efficiency of collecting viruses or microbes. Furthermore, the photocatalytic apatite 334 has the function of removing viruses or microbes. Accordingly, the air purifier 1 can reduce the generation of unpleasant odor or the occurrence of air contamination without the need of frequent cleaning or replacement.

(7)

In the air purifier 1, the photocatalytic apatite 334 having photocatalytic activity is carried on the surface of the photocatalytic filter 331 that is located downstream in the air flow. That is, air containing viruses, fungi or bacteria that have not adhered to the photocatalytic filter 331 comes in contact with the photocatalytic apatite 334. As a result, these viruses, fungi or bacteria are adsorbed in the photocatalytic apatite 334, and the photocatalytic apatite 334 removes them. That is, it is possible to reduce the possibility that ultraviolet radiation that is projected from the inverter lamps 35 onto the photocatalytic apatite 334 is blocked by the dust and dirt particles or the like adhering to the photocatalytic filter 331 when the photocatalytic apatite 334 removes the viruses, fungi or bacteria. Accordingly, since light that is sufficient to remove viruses or microbes is projected onto the photocatalytic apatite 334, the photocatalytic apatite 334 can remove even more viruses, fungi or bacteria. Consequently, the air purifier 1 can further reduce the generation of unpleasant odor or the occurrence of air contamination.

(8)

In the air purifier 1, the catechin 313 is carried on the surface of the fiber that constitutes the net portion 310 of the pre-filter 31. Catechin is generally known to have an excellent effect of inactivating viruses or inhibiting the proliferation of microbes. Accordingly, it is possible to further inhibit the proliferation of the fungi or bacteria adhering to the net portion 310, or to efficiently inactivate the viruses. Consequently, the air purifier 1 can further reduce the generation of unpleasant odor or the occurrence of air contamination.

(9)

In the air purifier 1, the catechin 313 is mixed into PP that forms the net portion 310 and thus is carried on the surface of the PP. Therefore, the catechin 313 is difficult to detach from the net portion 310. Accordingly, even if, for example, the user of the air purifier 1 washes the pre-filter 31 in order to clean the pre-filter 31, the catechin 313 will hardly detach from the net portion 310.

<Other embodiments>

Although the present invention has been described hereinabove, specific configurations of the invention are not limited to the above-described embodiment, and changes may be made without departing from the subject matter of the present invention.

(A)

In the above-described embodiment, the catechin 313 is carried on the net portion 310 of the pre-filter 31. In place of this (or in addition to this), a component of Yaku-sugi bogwood or a lytic enzyme may be carried on the net portion of the pre-filter. Components of Yaku-sugi bogwood are generally known to have an excellent effect of inhibiting the proliferation of microbes. In addition, lytic enzymes are generally known to have an excellent effect of inhibiting the proliferation of microbes since they dissolve the cell walls of microbes. Accordingly, the above-described air purifier can further inhibit the proliferation of microbes, thus further reducing the generation of unpleasant odor or the occurrence of air contamination.

(B)

In the above-described embodiment, the catechin 313 is carried on the net portion 310 of the pre-filter 31. In addition to this, the catechin may be carried on a titanium oxide filter or the second photocatalytic filter.

(C)

In the above-described embodiment, the apatite 334 is carried on the photocatalytic filter 331 of the first photocatalytic filter 33. In addition to this, the apatite may be carried on the net portion 310 of the pre-filter 31.

(D)

In the above-described embodiment, a visible light photocatalyst is carried on the net portion 310 of the pre-filter 31. However, in place of this, a mixture of a visible light photocatalyst and a photocatalytic apatite may be carried on the net portion 310 of the pre-filter 31.

Although apatite-based photocatalysts have higher adsorption ability with respect to viruses or microbes than conventionally used ones such as titanium oxide, they may not be able to obtain sufficient reactivity depending on the conditions of the light source. On the other hand, in the case of commonly used photocatalytic materials such as metal oxide photocatalysts or carbon-based photocatalysts, the dominant wavelength region of light in which catalytic reaction takes place can be easily changed, for example, by control of the crystal structure or by ion injection, so that they exhibit relatively high reactivity, regardless of the conditions of the light source. Accordingly, such a mixed photocatalyst can remove viruses or microbes that are adsorbed by apatite located in the vicinity of a commonly used photocatalyst such as a metal oxide photocatalyst or a carbon-based photocatalyst, even if the light source is in poor conditions.

(E)

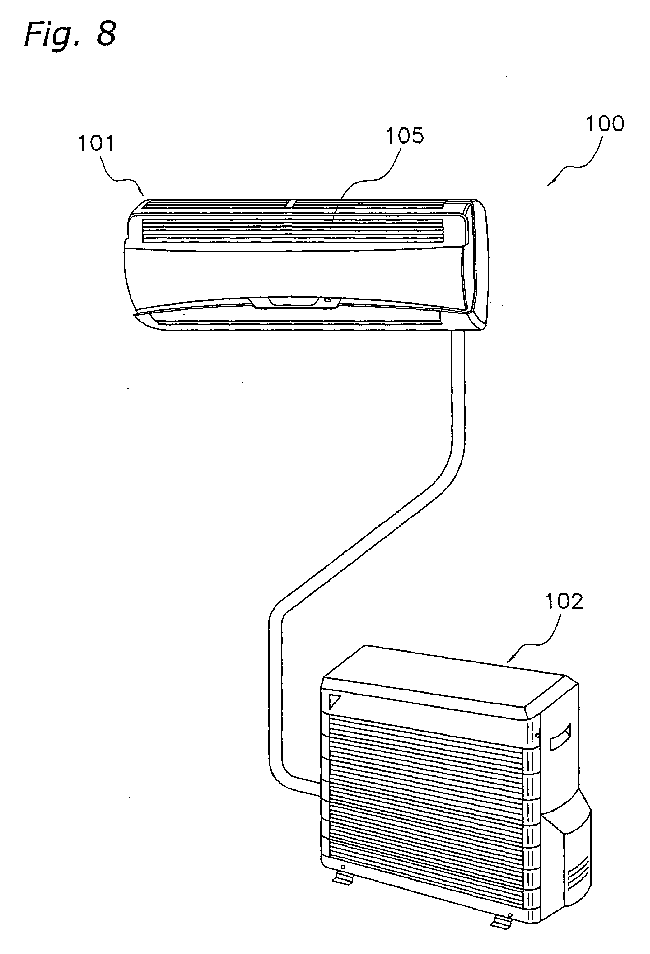

Although the above-described embodiment of the present invention is applied to the air purifier 1, the present invention also may be applied to an air conditioner 100 as shown in FIG. 8 that performs cooling and heating.

The air conditioner 100 is an apparatus for supplying conditioned air into a room, and includes an indoor device 101 that is installed, for example, on the wall in the room and an outdoor device that is installed outside the room. The indoor device 101 is provided with an intake port 105 for drawing indoor air into the air conditioner 100, and a filter unit (not shown) is provided on the inner side of the intake port 105. When the present invention is applied to this filter unit, it is also possible to reduce the generation of unpleasant odor or the occurrence of air contamination, since viruses, fungi or bacteria adhering to or adsorbed by the filter unit can be removed.

(F)

In the above-described embodiment, a photocatalytic apatite in which the photocatalytic apatite 334 is used as the apatite 334 is carried on the surface of the photocatalytic filter 331 that is located downstream in the air flow. In place of this, titanium oxide and apatite having photocatalytic activity may be carried on the surface of the photocatalytic filter 331 that is located downstream in the air flow.

(G)

Although in the above-described embodiment the plasma ionization portion 32 and the first catalytic filter 33 are provided separately from each other, the plasma ionization portion 32 and the first catalytic filter 33 may be formed as one unit in advance.

(H)

Although in the above-described embodiment the inverter lamps 35 were provided downstream of the first photocatalytic filter 33, the inverter lamps 35 may be provided upstream of the first photocatalytic filter 33. In general, dust or dirt particles to which viruses or microbes adhere are collected more on the upstream surface of the filter. This configuration, therefore, makes it possible to remove viruses or microbes with higher efficiency.

(I)

Although in the above-described embodiment the first photocatalytic filter 33 and the second photocatalytic filter are used as the air purification member, it is also possible to use, in place of this, an electric dust collector or the like, or an electret (an electrostatic filter (a filter in which substances having a positive charge and a negative charge are carried on fiber constituting unwoven fabric), for example. In the case of using an electric dust collector, an apatite layer may be provided in the collection electrode.

(J)

Although in the above-described embodiment the first photocatalytic filter 33 and the second photocatalytic filter are used as the air purification member, it is also possible to use, for example, a filter that carries a positive charge, in place of this. In general, viruses or microbes carry a negative charge, and it is therefore possible to actively collect viruses or microbes even if the plasma ionization portion 32 is not provided.

*Second embodiment*

<Overall configuration of air purification system>

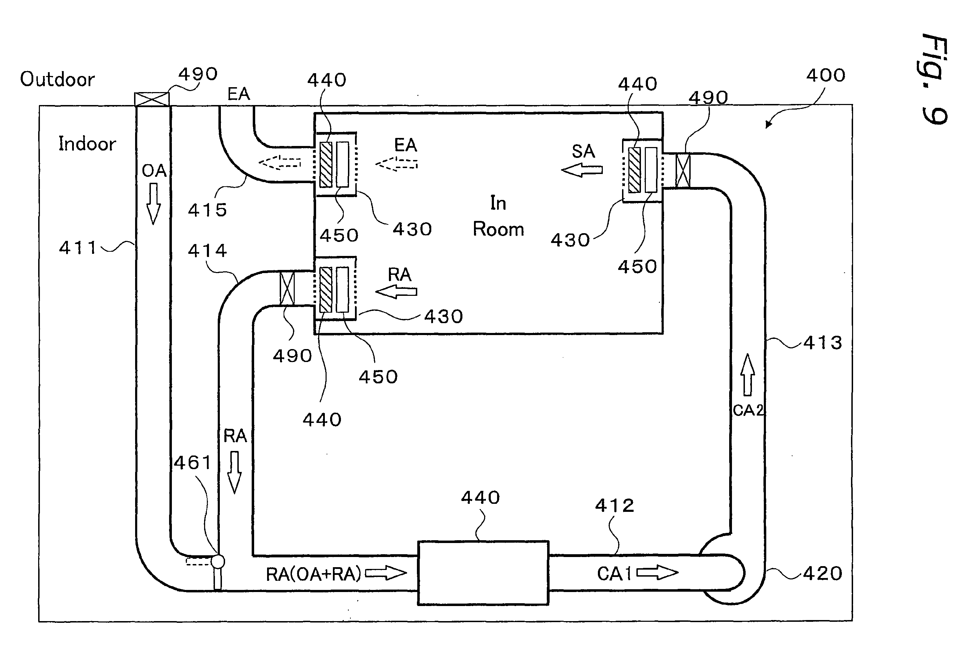

FIG. 9 is a diagram showing the system configuration of an air conditioning system 400 for which one embodiment of the present invention is employed.
The air conditioning system 400 is an air conditioning system for hospital use, and usually maintains the suspended dust concentration at 0.15 mg/m$^3$ or lower in a room. As shown in FIG. 9, the air conditioning system 400 is mainly constituted by an outside air inlet duct 411, an outside air inlet damper 461, a first duct 412, a duct type air conditioning unit 440, a blower 420, a second duct 413, air purification filter units 430, a third duct 414 and an exhaust duct 415.

<Structural components of air conditioning system>

(1) Outside air inlet duct

The outside air inlet duct 411 leads to the outside, and is provided for introducing outside air OA into the room. Additionally, one end of the outside air inlet duct 411 faces the outside, and a pre-filter 490 is provided at that end. The pre-filter 490 is a filter for removing relatively large dust or dirt particles. The other end of the outside air inlet duct 411 is connected by piping to the first duct 412 and the third duct 414. Further, the outside air inlet damper 461 is provided at that connection point.

(2) Outside air inlet damper

The outside air inlet damper 461 is provided at the connection point between the outside air inlet duct 411 and the first duct 412. The outside air inlet damper 461 can be switched between a first state and a second state. In the first state (the state indicated by the solid line), the introduction of outside air is interrupted. In the second state (the state indicated by the dotted line), the introduction of air is carried out. Accordingly, the main air flow in the first state is as follows: RA→CA1→CA2→SA→RA (see the white arrows in FIG. 9). On the other hand, the main air flow in the second state is as follows:

RA+OA→CA1→CA2→SA→RA→RA+OA (see the white arrows in FIG. 9).

(3) First duct

One end of the first duct 412 is connected by piping to the outside air inlet duct 411 and the third duct 414, and the other end is connected by piping to the entrance of the blower 420. Additionally, the duct type air conditioning unit 440 is provided in the middle of the first duct 412. It should be noted that mixed air of return air RA and indoor air, or mixed air of return air RA, outside air OA and indoor air is supplied to the duct type air conditioning unit 440.

(4) Duct type air conditioning unit

The duct type air conditioning unit 440 is provided in the middle of the first duct 412, and includes therein a blowing fan and a heat exchanger (both not shown). At the time of introducing outside air, the blowing fan draws in outside air through the outside air inlet duct 411 and the first duct 412. The blowing fan also draws in indoor air, even when outside air is introduced. Further, the blowing fan also draws in return air RA from the room, even when outside air is introduced. Then, the blowing fan supplies the drawn air to the blower 420. The heat exchanger is connected to an outdoor unit (not shown) via a refrigerant pipe. A refrigerant (a refrigerant liquid at the time of cooling, a refrigerant gas at the time of heating) is supplied to this heat exchanger from the outdoor unit via the refrigerant pipe. Then, in this heat exchanger, air is cooled or heated through heat exchange with the refrigerant, and thereby conditioned air CA1 is generated.

(5) Blower

The blower 420 is mainly constituted by a blowing fan and a fan motor (both not shown). The fan motor drives the blowing fan. Then, the blowing fan generates an air flow (see the white arrow CA2 in FIG. 9). It should be noted that this blowing fan delivers conditioned air CA2 into the room through the second duct 413.

(6) Second duct

One end of the second duct 413 is connected by piping to the exit of the blower 420, and the other end is connected to the inside of the room. Additionally, the pre-filter 490 is provided on the indoor side of the second duct. In the second duct 413, conditioned air is flown into the room by the blower 420 (see the white arrow CA2 in FIG. 9).

(7) Air purification filter unit

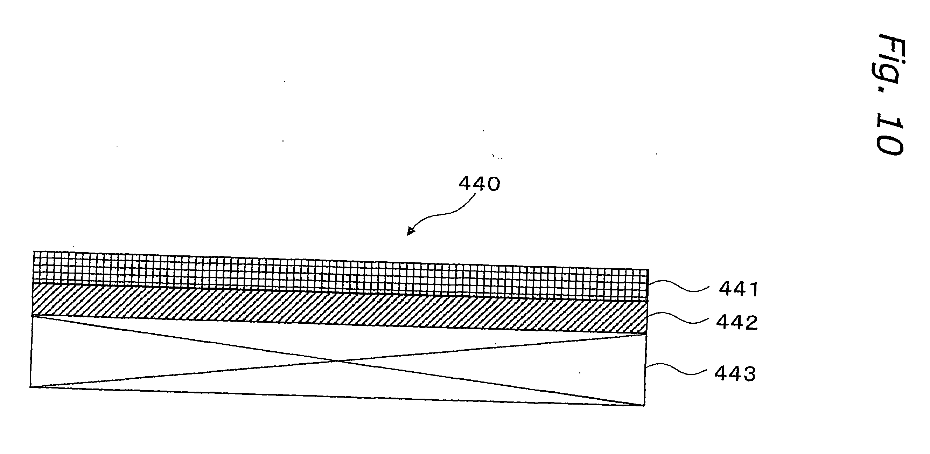

The air purification filter units 430 are provided at the indoor discharge port of the second duct 413, the indoor exhaust port of the third duct 414 and the indoor exhaust port of the fourth duct 415, respectively. Each of the air purification filter units 430 is mainly constituted by an advanced HEPA filter 440 and a corona discharge device 450. As shown in FIG. 10, a HEPA filter 443, an ozone decomposition catalyst layer 442 and a photocatalytic apatite layer 441 are provided in the advanced HEPA filter 440. The HEPA 443 is a filter having the performance to remove 99.97% or more of all kinds of particulates having a size of 0.3 μm or larger, whether they are dust, pollen or bacteria. The ozone decomposition catalyst layer is used for decomposing ozone that is generated by the corona discharge device 450. The photocatalytic apatite layer 441 is made of a substance in which a portion of the calcium atoms of calcium hydroxyapatite is replaced by titanium atoms, and exhibits high adsorption ability with respect to viruses or microbes, while functioning as a photocatalyst. It should be noted that each of the air purification filter units 430 is disposed such that the corona discharge device 450 faces upstream in the air flow and the advanced HEPA filter 440 faces downstream in the air flow.

(8) Third duct

One end of the third duct 414 is connected by piping to the first duct 412, and the other end is connected by piping to the indoor exhaust port. In the third duct 414, the air flows from the room to the duct type air conditioning unit 440. Additionally, the pre-filter 490 is provided on the indoor side of the third duct 414.

(9) Exhaust duct

One end of the exhaust duct 415 is connected by piping to the indoor exhaust port, and the other end leads to the outside. In this exhaust duct, a portion of air SA, which has been blown into the room, is exhausted (see the dotted white arrow EA in FIG. 9).

<Features of air conditioning system>

In the air conditioning system 400 according to the second embodiment, the air purification filter unit 430 is installed such that the corona discharge device 450 faces upstream in the direction of air flow and the advanced HEPA filter 440 faces downstream in the direction of air flow. Therefore, viruses or microbes are strongly charged in the corona discharge device 450 before they reach the photocatalytic apatite layer 441. Accordingly, more viruses or microbes can be adsorbed by the photocatalytic apatite layer 441. As a result, it is possible to improve the ability of the advanced HEPA filter 440 to collect viruses or microbes. Further, the photocatalytic apatite layer 441 of the advanced HEPA filter 440 is activated by the ultraviolet radiation generated by the above-described discharge. It is therefore not necessary to dispose a special light source in the air purification system 400. Consequently, it is possible to save the cost required for the light source.

<Other embodiments>

(A)

Although in the second embodiment the photocatalytic apatite layer 441 is provided in the advanced HEPA filter 440, it is possible to provide, in place of this, a layered mixture of apatite and a photocatalyst such as titanium dioxide, strontium titanate, zinc oxide, tungsten oxide, iron oxide, fullerene, nitride or oxynitride.

(B)

Although in the second embodiment the photocatalytic apatite layer 441 is provided in the advanced HEPA filter 440, it is possible to provide an apatite layer in place of this. In this case, although viruses or microbes are not actively removed, the collection ability is expected to be better than that of the HEPA filter.

(C)

Although in the second embodiment a HEPA filter is used as the advanced HEPA filter 440, it is possible to use a ULPA filter in place of this.

(D)

Although the second embodiment uses the corona discharge device 450, it is possible to use a plasma discharge device in place of this.

(E)

Although in the second embodiment the air purification filter units 430 are provided at the indoor discharge port of the second duct 413, the indoor exhaust port of the third duct 414 and the indoor exhaust port of the fourth duct 415, respectively, it is possible to provide, in addition to this, a further air purification filter unit 430 on the downstream side of the heat exchanger of the duct type air conditioning unit 440 in the direction of air flow. Usually, in an air conditioning apparatus, relative humidity tends to be low on the downstream side of the heat exchanger, regardless of whether the air conditioning apparatus is used for heating or for cooling. Basically, viruses, including SARS, tend to favor low-humidity environments. Accordingly, it is possible to collect viruses efficiently.

*Third embodiment*

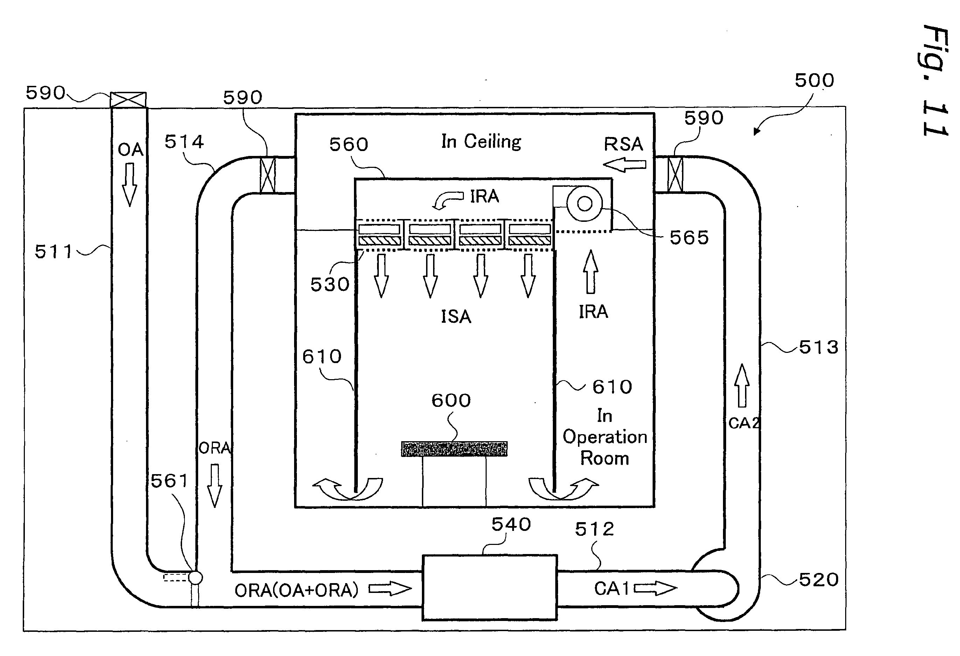

FIG. 11 is a diagram showing the system configuration of an air conditioning system 500 in which one embodiment of the present invention is employed.

<Overall configuration of air purification system>

FIG. 11 is a diagram showing the system configuration of an air conditioning system 500 in which one embodiment of the present invention is employed.

The air conditioning system 500 is an air conditioning system for hospital use, and usually maintains the suspended dust concentration at 0.15 mg/m$^3$ or lower in an operation room. As shown in FIG. 11, the air conditioning system 500 is mainly constituted by an outside air inlet duct 511, an outside air inlet damper 561, a first duct 512, a duct type air conditioning unit 540, a duct blower 520, a second duct 513, air purification units 560 and a third duct 514.

<Structural components of air conditioning system>

(1) Outside air inlet duct

The outside air inlet duct 511 leads to the outside, and is provided for introducing air OA from the outside into the room. Additionally, one end of the outside air inlet duct 511 faces the outside, and a pre-filter 590 is provided at that end. The pre-filter 590 is a filter for removing relatively large dust or dirt particles. The other end of the outside air inlet duct 511 is connected by piping to the first duct 512 and the third duct 514. Further, the outside air inlet damper 561 is provided at that connection point.

(2) Outside air inlet damper

The outside air inlet damper 561 is provided at the connection point between the outside air inlet duct 511 and the first duct 512. The outside air inlet damper 561 can be switched between a first state and a second state. In the first state (the state indicated by the solid line), the introduction of outside air is interrupted. In the second state (the state indicated by the dotted line), the introduction of air is carried out. Accordingly, the main air flow in the first state is as follows: ORA→CA1→CA2→RSA→ORA (see the white arrows in FIG. 11). On the other hand, the main air flow in the second state is as follows: ORA +OA→CA1→CA2→RSA→ORA→ORA+OA (see the white arrows in FIG. 11).

(3) First duct

One end of the first duct 512 is connected by piping to the outside air inlet duct 511 and the third duct 514, and the other end is connected by piping to the entrance of the duct blower 520. Additionally, the duct type air conditioning unit 540 is provided in the middle of the first duct 512. It should be noted that mixed air of return air RA and indoor air, or mixed air of return air RA, outside air OA and indoor air is supplied to the duct type air conditioning unit 540.

(4) Duct type air conditioning unit

The duct type air conditioning unit 540 is provided in the middle of the first duct 512, and includes therein a blowing fan and a heat exchanger (both not shown). At the time of introducing outside air, the blowing fan draws in outside air through the outside air inlet duct 511 and the first duct 112. The blowing fan also draws in indoor air, even when outside air is introduced. Further, the blowing fan also draws in return air RA from the room, even when outside air is introduced. Then, the blowing fan supplies the drawn air to the duct blower 520. The heat exchanger is connected to an outdoor unit (not shown) via a refrigerant pipe. A refrigerant (a refrigerant liquid at the time of cooling, a refrigerant gas at the time of heating) is supplied to this heat exchanger from the outdoor unit via the refrigerant pipe. Then, in this heat exchanger, air is cooled or heated through heat exchange with the refrigerant, and thereby conditioned air CA1 is generated.

(5) Duct blower

The duct blower 520 is mainly constituted by a blowing fan and a fan motor (both not shown). The fan motor drives the blowing fan. Then, the blowing fan generates air flow (see the white arrow CA2 in FIG. 11). It should be noted that this blowing fan delivers conditioned air CA2 into the room through the second duct 513.

(6) Second duct

One end of the second duct 513 is connected by piping to the exit of the duct blower 520, and the other end is connected to the inside of the room. Additionally, the pre-filter 590 is provided on the indoor side of the second duct. In the second duct 513, conditioned air is flown into the room by the duct blower 520 (see the white arrow CA2 in FIG. 11).

(7) Air purification unit

As shown in FIG. 11, the air purification unit 560 is placed in the ceiling, and is mainly constituted by an indoor blower 565 and an air purification filter unit 530. The indoor blowing fan draws in conditioned air RSA, which has been blown into the operating room, or return air IRA, which is in the operating room, and supplies that air to the air purification filter unit 530. The air that has passed through the air purification filter unit 530 flows perpendicularly to an operating table 600 and the floor surface in a glass screen 610 (see the white arrow ISA in FIG. 11). In addition, the main air flow in the operation room is as follows: RSA→IRA→ISA→(ORA or IRA) (see the white arrows in FIG. 11). The air purification filter unit 530 is identical to the air purification filter unit according to the second embodiment (see FIG. 10). It should be noted that the air purification unit 560 is installed such that the corona discharge device 550 faces upstream in the direction of the air flow and the advanced HEPA filter 540 faces downstream in the direction of the air flow.

(8) Third duct

One end of the third duct 514 is connected by piping to the first duct 512, and the other end is connected by piping to the indoor exhaust port. In the third duct 514, the air flows from the room to the duct type air conditioning unit 540. Additionally, the pre-filter 590 is provided on the indoor side of the third duct 514.

[Features of air conditioning system]

In the air conditioning system 500 according to the third embodiment, the air purification filter unit 530 is installed such that the corona discharge device faces upstream in the direction of air flow and the advanced HEPA filter faces downstream in the direction of air flow. Therefore, viruses or microbes are strongly charged in the corona discharge device before they reach the photocatalytic apatite layer 541. Accordingly, more viruses or microbes can be adsorbed by the photocatalytic apatite layer 541. As a result, it is possible to improve the ability of the advanced HEPA filter to collect viruses or microbes. Further, the photocatalytic apatite layer of the advanced HEPA filter is activated by the ultraviolet radiation generated by the above-described discharge. It is therefore not necessary to dispose a special light source in the air purification system 500. Consequently, it is possible to save the cost required for the light source.

<Other embodiments>

(A)

Although in the third embodiment the photocatalytic apatite layer 441 is provided in the advanced HEPA filter 440, it is possible to provide, in place of this, a layered mixture of apatite and a photocatalyst such as titanium dioxide, strontium titanate, zinc oxide, tungsten oxide, iron oxide, fullerene, nitride or oxynitride.

(B)

Although in the third embodiment the photocatalytic apatite layer 441 and the ozone decomposition catalyst layer 442 are provided in the advanced HEPA filter 440, it is possible to provide only an apatite layer.

(C)

Although in the third embodiment a HEPA filter is used as the advanced HEPA filter 440, it is possible to use a ULPA filter in place of this.

(D)

Although the third embodiment uses the corona discharge device 450, it is possible to use a plasma discharge device in place of this.

Industrial Applicability

By using the present invention, it is possible to reduce the generation of unpleasant odor or the occurrence of air contamination without the need of frequent cleaning or replacement.

**Claims**

1. An air purification member (31, 33, 440) for purifying air that contains particulates containing viruses or microbes, comprising:

   a dust collection portion (310, 330, 443) for collecting the particulates; and
   a microbe removal portion (312, 334, 441) for removing the viruses or the microbes that are collected by the dust collection portion (310, 330, 443).

2. The air purification member (31, 33, 340) according to claim 1,
   wherein the dust collection portion (310, 330, 443) carries thereon the microbe removal portion (312, 334, 441).

3. The air purification member (31, 33) according to claim 2,
   wherein the microbe removal portion (312, 334) includes a photocatalyst.

4. The air purification member (31, 33) according to claim 1,
   wherein the microbe removal portion includes a photocatalyst that is included in the dust collection portion (310, 330).

5. The air purification member (31) according to claim 3 or 4,
   wherein the photocatalyst (312) is a visible light photocatalyst.

6. The air purification member (33, 440) according to claim 3 or 4,
   wherein the photocatalyst (334, 441) is apatite having photocatalytic activity.

7. The air purification member (31, 33) according to any of claims 3 to 5,
   wherein the photocatalyst is a mixed photocatalyst in which the apatite having photocatalytic activity and a photocatalytic material are mixed.

8. The air purification member (31) according to any of claims 3 to 7,
   wherein the dust collection portion (310) is formed by fiber that is constituted by a core (310a) and a covering layer (314) covering the core and holding the photocatalyst (312) in such a manner that a portion of the photocatalyst (312) is exposed to the air side.

9. The air purification member (33) according to claim 4 or 5,
   wherein the apatite (334) is included in the dust collection portion (331).

10. The air purification member according to claim 9,
    wherein the apatite is provided on an upstream surface of the dust collection portion with respect to a direction of air flow.

11. The air purification member (33) according to claim 9,
    wherein a light source is disposed in a space located downstream of the dust collection portion (330), and
    wherein the apatite (334) and the photocatalyst are provided on a downstream surface of the dust collection portion (330) with respect to a direction of air flow.

12. The air purification member (31) according to any of claims 1 to 11, further comprising:

    an antimicrobial portion for inactivating the viruses or inhibiting proliferation of the microbes.

13. The air purification member (31) according to claim 12,

wherein the antimicrobial portion is carried on the dust collection portion (310).

14. The air purification member (31) according to claim 12 or 13,
    wherein the antimicrobial portion includes catechin (313).

15. The air purification member according to any of claims 12 to 14, wherein the antimicrobial portion releases a component of Yaku-sugi (Cryptomeria japonica) bogwood.

16. The air purification member according to any of claims 12 to 15, wherein the antimicrobial portion includes a lytic enzyme.

17. The air purification member according to any of claims 1 to 16,
    wherein the dust collection portion is positively charged.

18. The air purification member according to claim 3,
    wherein the dust collection portion is an electrode.

19. An air conditioning apparatus (1) for supplying conditioned air into a room, comprising:

    a casing (10);
    a blowing portion (20) for blowing air that is drawn into the casing (10) into the room; and
    the air purification member according to any of claims 1 to 18 through which the air drawn into the casing (10) passes through.

20. The air conditioning apparatus according to claim 19, further comprising:

    a dehumidification portion for dehumidifying the air,

    wherein the air purification member is disposed downstream of the dehumidification portion with respect to a direction of air flow.

21. An air purification unit (430) comprising:

    a charging portion (32, 450) for charging viruses or microbes that are contained in air; and
    an air purification portion (33, 440) that includes apatite (334, 441) for adsorbing the viruses or the microbes.

22. The air purification unit (430) according to claim 21,
    wherein the air purification portion (33, 440) further comprises a microbe removal portion (441) for removing the viruses or the microbes.

23. The air purification unit (430) according to claim 22,
    wherein the microbe removal portion (441) includes a photocatalyst.

24. The air purification unit (430) according to any of claims 21 to 23,
    wherein the charging portion (450) produces ultraviolet radiation by causing a discharge.

25. The air purification unit according to claim 23 or 24,
    wherein the air purification portion is an electrode.

26. An air conditioning apparatus (1) for supplying conditioned air into a room, comprising:

    a casing (10);
    a blowing portion (20) for blowing air that is drawn into the casing (10) into the room;
    a charging portion (32) for charging viruses or microbes that are contained in the air; and
    an air purification portion (33) that is provided downstream of the charging portion with respect to a direction of air flow, and that includes apatite (334) for adsorbing the viruses or the microbes and a microbe removal portion for removing the viruses or the microbes.

27. The air conditioning apparatus according to claim 26, further comprising:

   a dehumidification portion for dehumidifying the air,

   wherein the air purification member is disposed downstream of the dehumidification portion in the direction of air flow.

28. The air conditioning apparatus (1) according to claim 26 or 27,
   wherein the microbe removal portion is a photocatalyst.

29. The air conditioning apparatus (1) according to claim 28,
   wherein the apatite (334) and the photocatalyst are the same substance.

30. The air conditioning apparatus according to claim 28 or 29,
   wherein the charging portion produces ultraviolet radiation by causing a discharge.

31. The air conditioning apparatus according to any of claims 28 to 30,
   wherein the air purification portion is an electrode.

32. An air purification member (440) for purifying air that contains particulates containing viruses or microbes and that has a suspended dust concentration of 0.15 mg/m$^3$ or less, comprising:

   a HEPA filter (443); and
   apatite (441) provided in the HEPA filter (443) for adsorbing the viruses or the microbes.

33. The air purification member (440) according to claim 32,
   wherein the apatite (441) is provided on an upstream surface of the HEPA filter with respect to a direction of air flow.

34. The air purification member (440) according to claim 33, further comprising:

   a photocatalyst (441) provided on the upstream surface of the HEPA filter (443) in the direction of air flow.

35. The air purification member (440) according to claim 34, wherein the apatite and the photocatalyst are apatite (441) having photocatalytic activity, or a mixed photocatalyst in which apatite having photocatalytic activity and a photocatalytic material are mixed.

36. An air purification unit (430) comprising:

   the air purification member (440) according to claim 34 or 35; and
   a light source (450) disposed in a space located upstream of the HEPA filter (443) with respect to a direction of air flow.

## Fig. 1

Fig. 2

# Fig. 3

Display Panel 56

Fan Motor 21

Control Portion 50

ROM 51

RAM 52

Temperature Sensor 53

Humidity Sensor 54

Dust Sensor 55

Fig. 4

311

310

31

Fig. 5

# Fig. 6

Direction of Air Flow

33

331

330

334

# Fig. 7

Direction of Air Flow

33

331

330

334

336

336

Adherence

337

337

338

336

336

338

337

336

Adherence

337

338

338

Adherence

Fig. 8

# Fig. 9

# Fig. 10

Fig. 11

## INTERNATIONAL SEARCH REPORT

| International application No. |
|---|
| PCT/JP2004/002321 |

**A. CLASSIFICATION OF SUBJECT MATTER**
Int.Cl⁷ B01D46/10, 46/54, B03C3/02, 3/14, 3/45, A61L9/16, 9/00, 9/18, A01N25/34, 43/16, 59/16, 63/00, 65/00

According to International Patent Classification (IPC) or to both national classification and IPC

**B. FIELDS SEARCHED**

Minimum documentation searched (classification system followed by classification symbols)
Int.Cl⁷ B01D46/10-46/54, B03C3/02, 3/14, 3/28, 3/45-3/60, A61L9/16, 9/00, 9/18, A01N25/34, 43/16, 59/16, 63/00, 65/00

Documentation searched other than minimum documentation to the extent that such documents are included in the fields searched
Jitsuyo Shinan Koho                1926–1996    Toroku Jitsuyo Shinan Koho    1994–2004
Kokai Jitsuyo Shinan Koho    1971–2004    Jitsuyo Shinan Toroku Koho    1996–2004

Electronic data base consulted during the international search (name of data base and, where practicable, search terms used)
JICST FILE(JOIS)

**C. DOCUMENTS CONSIDERED TO BE RELEVANT**

| Category* | Citation of document, with indication, where appropriate, of the relevant passages | Relevant to claim No. |
|---|---|---|
| X/Y | JP 11-342310 A  (Mitsubishi Paper Mills Ltd.), 14 December, 1999 (14.12.99), Full text; particularly, Claim 9; page 5, Par. No. [0051]; page 8, Par. No. [0083]; page 13, Par. No. [0146]; Fig. 1 (Family: none) | 1-3,5,9, 11-12,17/ 6-7,10, 14-16,21-31 |
| X/Y | JP 10-33921 A  (Sekisui Plastics Co., Ltd.), 10 February, 1998 (10.02.98), Full text; particularly, page 3, Par. No. [0002]; page 4, Par. No. [0083] (Family: none) | 1-5,8,12-13, 19/6-7,10, 14-16,20 |

☒ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09 June, 2004 (09.06.04) | 06 July, 2004 (06.07.04) |

| Name and mailing address of the ISA/ | Authorized officer |
|---|---|
| Japanese Patent Office | |
| Facsimile No. | Telephone No. |

Form PCT/ISA/210 (second sheet) (January 2004)

| | **INTERNATIONAL SEARCH REPORT** | International application No. |
|---|---|---|
| | | PCT/JP2004/002321 |

C (Continuation). DOCUMENTS CONSIDERED TO BE RELEVANT

| Category* | Citation of document, with indication, where appropriate, of the relevant passages | Relevant to claim No. |
|---|---|---|
| X<br>/Y | JP 2001-254245 A (Gifu-Ken, Kabushiki Kaisha Hasetora Shoten, Gisen Kabushiki Kaisha, Takada Kasei Kogyo Kabushiki Kaisha, Zaidan Hojin Gifuken Kenkyu Kaihatsu Jigyodan), 21 September, 2001 (21.09.01), Full text; particularly, page 3, Par. No. [0013] (Family: none) | 1-5,9-10,19<br>/14-16,20 |
| Y | JP 2000-327315 A (Fujitsu Ltd.), 28 November, 2000 (28.11.00), Full text (Family: none) | 6-7,14-16, 29-31,34-36 |
| Y | JP 2001-302220 A (Fujitsu Ltd.), 31 October, 2001 (31.10.01), Full text (Family: none) | 6-7,14-16, 29-31,34-36 |
| Y | JP 2002-306911 A (Mitsubishi Paper Mills Ltd.), 22 October, 2002 (22.10.02), Full text; particularly, page 3, Par. No. [0023] (Family: none) | 14 |
| Y | JP 2002-28414 A (Mitsubishi Paper Mills Ltd.), 29 January, 2002 (29.01.02), Full text (Family: none) | 14 |
| Y | JP 2001-226827 A (ELP Corp.), 21 August, 2001 (21.08.01), Full text & TW 478938 B & CN 1297784 A & US 2001-0008690 A1 | 14 |
| Y | JP 2001-137629 A (Mitsubishi Paper Mills Ltd.), 22 May, 2001 (22.05.01), Full text; particularly, page 4, Par. No. [0034] (Family: none) | 14 |
| Y | JP 7-148407 A (Matsushita Seiko Co., Ltd.), 13 June, 1995 (13.06.95), Full text (Family: none) | 14 |
| Y | JP 6-40833 A (Kabushiki Kaisha Yamamuro), 15 February, 1994 (15.02.94), Full text (Family: none) | 15 |

Form PCT/ISA/210 (continuation of second sheet) (January 2004)

**EP 1 600 201 A1**

**INTERNATIONAL SEARCH REPORT**

International application No.

PCT/JP2004/002321

C (Continuation). DOCUMENTS CONSIDERED TO BE RELEVANT

| Category* | Citation of document, with indication, where appropriate, of the relevant passages | Relevant to claim No. |
|---|---|---|
| Y | Shin'ichi MORITA, "Mokushitsukei Shigen no Chushutsu Seibun Riyoka ni Kansuru Kenkyu Dai 1 Ho Yakusugi Tsuchi Maiboku Chushutsu Seibun no Teiryo to Hexan Chushutsubutsu no Satsudani Kokin Kassei", Reports of Kagoshima Prefectural Institute of Industrial Technology, 1990, Vol.3, pages 79 to 84 | 15 |
| Y | JP 6-91117 A (Ebara Corp.), 05 April, 1994 (05.04.94), Full text (Family: none) | 16 |
| Y | JP 10-309492 A (Daikin Industries, Ltd.), 24 November, 1998 (24.11.98), Full text (Family: none) | 18 |
| Y | JP 10-309493 A (Daikin Industries, Ltd.), 24 November, 1998 (24.11.98), Full text (Family: none) | 18 |
| Y | JP 10-328575 A (Toyo Element Kogyo Kabushiki Kaisha), 15 December, 1998 (15.12.98), Full text (Family: none) | 18,25,31 |
| Y | JP 10-61986 A (Daikin Industries, Ltd.), 06 March, 1998 (06.03.98), Full text; particularly, Claim 1; page 4, Par. Nos. [0016], [0019];Fig. 1 (Family: none) | 21-24,26, 28-30 |
| Y | JP 5-68820 A (Bestex Kabushiki Kaisha), 23 March, 1993 (23.03.93), Full text; particularly, Claim 1; page 2, Par. No. [0001]; page 4, Par. Nos. [0029] to [0030] & EP 497594 A1 | 21-23 |
| Y | JP 2002-253658 A (Toshiba Corp.), 10 September, 2002 (10.09.02), Full text (Family: none) | 24,30 |
| Y | JP 2002-79016 A (Mitsubishi Paper Mills Ltd.), 19 March, 2002 (19.03.02), Full text; particularly, page 3, Par. No. [0024]; page 4, Par. Nos. [0033], [0035] (Family: none) | 32-36 |

Form PCT/ISA/210 (continuation of second sheet) (January 2004)

39

| | **INTERNATIONAL SEARCH REPORT** | International application No.<br>PCT/JP2004/002321 |
|---|---|---|

**C (Continuation).** DOCUMENTS CONSIDERED TO BE RELEVANT

| Category* | Citation of document, with indication, where appropriate, of the relevant passages | Relevant to claim No. |
|---|---|---|
| Y | JP 2002-95910 A  (Mitsubishi  Paper Mills Ltd.),<br>02 April, 2002 (02.04.02),<br>Full text; particularly, page 2, Par. No. [0012];<br>page 4, Par. No. [0028]<br>(Family: none) | 34-35 |
| Y | JP 2001-336813 A  (Saburo OOHARA, Arutan<br>Kabushiki Kaisha),<br>07 December, 2001 (07.12.01),<br>Full text<br>(Family: none) | 34-35 |
| Y | JP 11-221442 A  (Sharp Corp.),<br>17 August, 1999 (17.08.99),<br>Full text; particularly, page 3, Par. No. [0017];<br>page 5, Par. No. [0027]; Fig. 4<br>(Family: none) | 34-36 |

Form PCT/ISA/210 (continuation of second sheet) (January 2004)

**INTERNATIONAL SEARCH REPORT**

| International application No. |
|---|
| PCT/JP2004/002321 |

**Box No. II**     **Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III**     **Observations where unity of invention is lacking (Continuation of item 3 of first sheet)**

This International Searching Authority found multiple inventions in this international application, as follows:

    (See extra sheet.)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**     ☐ The additional search fees were accompanied by the applicant's protest.

☐ No protest accompanied the payment of additional search fees.

Form PCT/ISA/210 (continuation of first sheet (2)) (January 2004)

**INTERNATIONAL SEARCH REPORT**

International application No.

PCT/JP2004/002321

Continuation of Box No.III of continuation of first sheet(2)

Whether or not independent claims 1, 21, 26 and 32 not quoting any other claims satisfy the requirement of unity of invention will be studied.

The technical feature of the invention of claim 1 is an air cleaning member comprising a dust collection part capable of trapping minute particles with viruses or bacteria and a microorganism eliminating part. The technical feature of the invention of claim 21 is to comprise an electrification part for electrifying viruses or bacteria and an air cleaning part including an apatite capable of adsorbing viruses or bacteria. The technical feature of the invention of claim 26 is to comprise an electrification part and an air cleaning part which includes apatite and a microorganism eliminating part. The technical feature of the invention of claim 32 is an air cleaning member comprising an HEPA filter and an apatite disposed on the HEPA filter. The inventions of claims 21 and 32 do not include a "microorganism eliminating part" which is one of the technical features of first claimed invention (main invention). Thus, the matters common to all the inventions of claims 1, 21, 26 and 32 (referred to as common matters) are only limitation of treated air to air containing viruses or bacteria and having of an air cleaning member or an air cleaning part. These common matters are technical matters publicly known prior to the filing of the instant application without the need to indicate references and fall within the category of prior art. Consequently, these common matters cannot be "special technical features" within the meaning of PCT Rule 13.2, second sentence. Therefore, between the inventions of claims 1, 21, 26 and 32, there is no technical relationship involving one or more of the same or corresponding special technical features, and hence these inventions cannot be recognized as being linked with each other so as to form a single general inventive concept.

Form PCT/ISA/210 (extra sheet) (January 2004)